# EUROPEAN PATENT APPLICATION

(11) **EP 4 751 751 A2**
(43) Date of publication of application: **03.06.2026**
(21) Application number: 26169989.6
(22) Date of filing: 14.12.2021
(51) Int. Cl.: A61M 5/32

(54) **FLUID DELIVERY SYSTEM WITH NEEDLE ASSEMBLY**

(30) Priority: 17.12.2020 US 202063126552 P
(62) Divisional of application: 21840331.9
(71) Applicant: Eli Lilly and Company, Indianapolis, IN 46285 (US)
(72) Inventor: GUNAY, Murat, Indianapolis, 46206-6288 (US); JUDSON, Jared Alden, Indianapolis, 46206-6288 (US); PERKINS, Russell Wayne, Indianapolis, 46206-6288 (US); SCHAFF, Anthony Lawrence, Indianapolis, 46206-6288 (US)
(74) Representative: J A Kemp LLP

(57) **Abstract**

A needle assembly comprising: a first needle coupled to a first needle support; a second needle coupled to a second needle support; a movable connector coupling the first needle to the second needle; and a driving mechanism configured to rotate and drive the first needle support in a first direction, and to drive the second needle support in a second direction.

## Description

### FIELD OF THE DISCLOSURE

The present disclosure relates to a needle assembly to deliver a fluid. More specifically, the present disclosure relates to an extendable and retractable needle assembly configured to deliver a fluid through a surface.

### BACKGROUND OF THE DISCLOSURE

Conventional injection devices are often used to drive a needle through a surface, for example in the injection of a drug, removing a fluid from a sealed container such as a vial, sampling within chemical instrumentation, and so on. Considering the example of injecting a patient with a drug, it is sometimes advantageous for the drug to be administered without the presence of a medical professional (e.g. when taken frequently). It may be a challenge to ensure that the needle is maintained in a sterile environment prior to injection, as well to ensure that the drug is administered safely and efficiently. Furthermore, some patients are uncomfortable with seeing or directly handling needles.

### SUMMARY

A needle assembly configured to be part of a drug delivery device comprises a plurality of extendable and retractable needles. The needles are fluidly coupled by a flexible connector, which is configured to maintain fluid connection between the needles throughout their movement. When the needles are extended, a first needle pierces a patient's skin, and a second needle pierces a septum of a drug container. Once the drug has been delivered, the needles may then retract within a needle assembly housing. The needle assembly may also be part of a partially disposable drug delivery device and may be used with multiple different drug containers. Multiple embodiments of the needle assembly are disclosed.

In one embodiment, a drug delivery device includes a container containing a medication, and a needle assembly coupled to the container. The needle assembly includes a housing with a first end and a second end configured to interface with the container. A first needle support and a second needle support are positioned within the housing. A first needle is coupled to the first needle support; a second needle is coupled to the second needle support. A movable connector is in fluid communication between the first and second needles. A driving mechanism is configured to move the needle assembly from a first configuration to a second configuration. In the first configuration, the first needle and the second needle are positioned completely within the housing. In the second configuration, the first needle extends beyond the first end of the housing and the second needle extends beyond the second end of the housing. A delivery mechanism is coupled to the container and configured to drive the medication through the second needle and the first needle when the needle assembly is in the second configuration.

In another embodiment, a needle assembly includes a housing configured to couple to a fluid delivery mechanism and to interface with a surface. A first needle is coupled to a first needle support, and a second needle is coupled to second needle support. A movable connector couples the first needle to the second needle. A driving mechanism is supported by the housing and configured to drive the first needle support in a first direction, and to drive the second needle support in a second direction.

In yet another embodiment, a method of operation of a device includes the steps of: Activating a first driving mechanism of the device a first time, wherein the first driving mechanism moves a first needle in a first direction from a retracted configuration to an axially extended configuration, and moves a second needle in a second direction towards a septum of a drug container. Activating a second driving mechanism, wherein the second driving mechanism drives a medication through the second needle out of and the first needle. Activating the first driving mechanism a second time subsequent to activating the second drive mechanism, wherein the first driving mechanism moves the first needle in the second direction from the axially extended configuration to the retracted configuration and moves the second needle in the first direction away from the septum.

In another embodiment, a needle assembly includes a housing configured to interface with a surface at a first end, and to interface with a container at a second end. A plurality of needle mechanisms each comprising a first needle fluidly coupled to a second needle. At least one driving mechanism is configured to engage at least one of the needle mechanisms wherein the first needle is driven in a first direction and the second needle is driven in a second direction approximately opposite to the first direction. A rotating mechanism is configured to rotate the plurality of needle mechanisms within the housing.

In yet another embodiment, a drug delivery device includes a housing, at least one container within the housing configured to retain a fluid, and a first motor configured to drive the fluid from the container with variable force. A needle assembly is configured to move from a first configuration to a second configuration. In the first configuration, the needle assembly is retracted, and in the second configuration, the needle assembly is extended and in fluid communication with the container.

### BRIEF DESCRIPTION OF THE DRAWINGS

The above-mentioned and other features and advantages of this disclosure, and the manner of attaining them, will become more apparent and the invention itself will be better understood by reference to the following description of embodiments of the invention taken in conjunction with the accompanying drawings, wherein:
FIG. 1 is a simplified, cross-sectional view of an exemplary drug delivery device;
FIG. 2 is a simplified, cross-sectional view of another exemplary drug device with a detachable cassette and needle assembly;
FIG. 3 is a simplified, cross-sectional view of yet another exemplary drug delivery device with a detachable needle assembly;
FIG .4 is a side elevational view of an exemplary drug delivery device having a cassette and a driving module;
FIG. 5 is a vertical cross section of the device of FIG. 4;
FIG. 6 is a cross section taken along line B-B of FIG. 5;
FIG. 7 is a cross section taken along line C-C of FIG. 5;
FIGS. 8-10 are exploded views of the device of FIG. 4 showing the driving module and the cassette separated;
FIG. 11 is a perspective view of another embodiment of an exemplary drug delivery device having a cassette and a needle assembly;
FIG. 12 is an exploded view of the device of FIG. 11;
FIG. 13 is a perspective view of a cartridge from the device of FIG. 11;
FIG. 14 is a perspective view of a driving mechanism of the device of FIG. 11;
FIG. 15 is a diagrammatic view of the driving mechanism of FIG. 14;
FIG. 16 is a diagrammatic view of a control system for the driving mechanism of FIG. 14;
FIG. 17 is a perspective view of an exemplary needle assembly;
FIG. 18 is a rear view of the needle assembly of FIG. 17;
FIG. 19 is an exploded view of the needle assembly of FIG. 17;
FIG. 20 is a cross-sectional view of the needle assembly of FIG. 17;
FIGS. 21 and 22 are perspective views of a needle mechanism within the needle assembly of FIG. 17;
FIGS. 23 and 24 are side elevational views of the needle mechanism of FIGS. 21 and 22 without the driving member;
FIGS. 25 and 26 are perspective views of a driving member of the needle mechanism of FIGS. 21 and 22;
FIGS 27 and 28 are side views of the needle mechanism of FIGS. 21 and 22 showing a motion of the needle mechanism from a first configuration in FIG. 27 to a second configuration in FIG. 28;
FIG. 29 is a perspective view of another embodiment of a needle assembly;
FIG. 30 is a rear view of the needle assembly of FIG. 29;
FIG. 31 is an exploded view of the needle assembly of FIG. 29;
FIG. 32 is a perspective view of a needle mechanism within the needle assembly of FIG. 29;
FIG. 33 is a perspective view of yet another embodiment of a needle assembly;
FIG. 34 is an exploded view of the needle assembly of FIG. 33;
FIG. 35 is a perspective view of a needle mechanism within the needle assembly of FIG. 33;
FIG. 36 is a perspective view of a needle support of the needle mechanism of FIG. 35;
FIG. 37 is a perspective view of a driving member of the needle mechanism of FIG. 35;
FIG. 38 and 39 are side views of a needle mechanism within the needle assembly of FIG. 33 showing a movement of the needle mechanism from a first configuration in FIG. 38 to a second configuration in FIG. 39;
FIGS. 40-41 are perspective views of yet another embodiment of a needle assembly in a first configuration and a second configuration respectively;
FIG. 42 is an exploded view of the needle assembly of FIGS. 40-41; and
FIG. 43 is a diagrammatic view of an exemplary wireless communication system for use with a drug delivery device.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

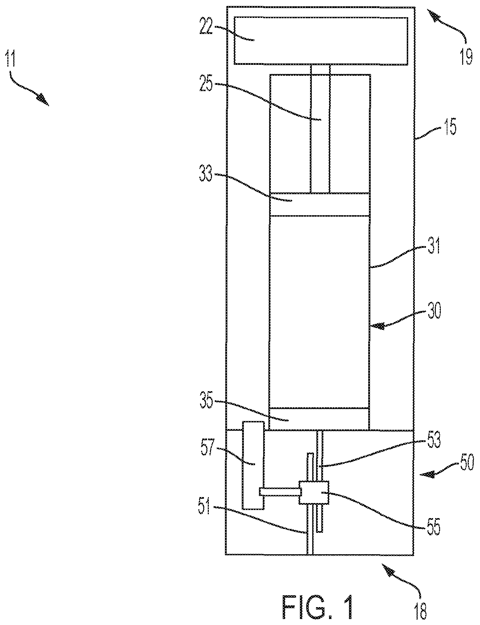

Exemplary drug delivery devices 11, 12, 13, 126, and 1000 are illustrated in FIGS. 1-4 and 11. Each of the shown devices 11, 12, 13, 126, and 1000 is a reusable pen-shaped medication injection device which may be manually handled by a user (e.g., a patient, a caregiver, or a healthcare professional) to deliver a medication to a patient. In certain embodiments, the user may selectively set a dose and then to inject that set dose into the patient. Devices 11, 12, 13, 126, and 1000 are intended to be illustrative and not limiting as the needle assemblies described further below may be used in other differently configured devices.

The medication may be any type that may be delivered by such a device 11, 12, 13, 126, and 1000. The medication may be in fluid form or any other suitable form. The medication includes one or more therapeutic agents including but not limited to insulins, insulin analogs such as insulin lispro or insulin glargine, insulin derivatives, GLP-1 receptor agonists such as dulaglutide or liraglutide, glucagon, glucagon analogs, glucagon derivatives, gastric inhibitory polypeptide (GIP), GIP analogs, GIP derivatives, dual agents of any combination above, such as, for example, GIP/GLP-1 receptor agonist, oxyntomodulin analogs, oxyntomodulin derivatives, therapeutic antibodies and any therapeutic agent that is capable of delivery by the device 11, 12, 13, 126, and 1000. The medication as used in the device 11, 12, 13, 126, and 1000 may be formulated with one or more excipients.

Referring first to FIG. 1, an exemplary embodiment of a drug delivery device 11 is shown. Drug delivery device 11 extends from a proximal end 19 to a distal end 18, and comprises a housing 15, a first motor 22, a driving system 25, a cartridge 30 configured to retain the medication, and a needle assembly 50. In the illustrated embodiment, drug delivery device 11 is configured to be positioned with distal end 18 near a patient's skin, such that needle assembly 50 may deliver the medication from within cartridge 30 to the patient. Cartridge 30 may also be described as a cassette, or a syringe, and is generally configured to contain and at least partially deliver the medication.

Cartridge 30 of FIG. 1 additionally comprises a fluid housing or barrel 31, a plunger or stopper 33, and a septum 35. The medication is retained within fluid housing 31 by stopper 33. The activation of first motor 22 actuates driving system 25 to drive stopper 33 downward to push the medication towards septum 35 and ultimately through needle assembly 50. First motor 22 may be controlled by a motor controller (not shown) to adjust the force applied to driving system 25 and/or the rate that driving system 25 is actuated.

Needle assembly 50 additionally comprises a first needle 51, a second needle 53, a needle driving mechanism 55 coupled to the first and second needles 51, 53, and an optional second motor 57 configured to actuate needle driving mechanism 55. Activation of second motor 57 actuates needle driving mechanism 55 and drives first needle 51 towards distal end 18 and drives second needle 53 towards proximal end 19 into an extended configuration whereby first needle 51 pierces at least the patient's skin, and second needle 53 pierces septum 35. In some embodiments, drug delivery device 11 only comprises first motor 22 and does not comprise the second motor 57. In such embodiments, first motor 22 may be coupled or linked to needle driving mechanism 55 such that activation of first motor 22 may additionally actuate needle driving mechanism 55. As will be described in more detail later, first needle 51 and second needle 53 are fluidly coupled together to allow flow of the medication from cartridge 30, through second needle 53, through first needle 51, and ultimately to the patient.

Drug delivery device 11 is configured as a singular device and may also be configured to be disposable after use. In the illustrated embodiment, cartridge 30 and needle assembly 50 are fixedly coupled to and/or an integral part of drug delivery device 11. Cartridge 30 may contain a single dose of the medication to be delivered or may contain the medication to be delivered in multiple doses/injections. In embodiments where cartridge 30 comprises multiple doses of the medication, first motor 22 may be configured to only push out a portion of the medication within cartridge 30 for each dose, and needle assembly 50 may comprise multiple sets of needles to inject the medication into a patient.

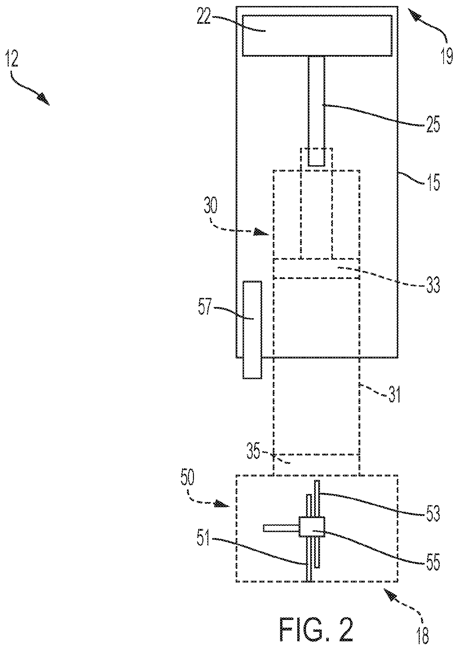

Referring now to FIG. 2, another embodiment of a drug delivery device 12 is shown. Drug delivery device 12 functions similar to drug delivery device 11 of FIG. 1 and comprises similar components. However, drug delivery device 12 differs from drug delivery device 11 in that cartridge 30 and needle assembly 50 are removable from housing 15. Needle assembly 50 and cartridge 30 may be separate components from one another or may be one single component. In the illustrated embodiment, housing 15, first motor 22, driving system 25, and second motor 57 may all be reusable, and cartridge 30 and needle assembly 50 may be disposable. In such an embodiment, a user may insert, use, and remove multiple instances of cartridge 30 and needle assembly 50, either together or separately. As was the case with drug delivery device 11, cartridge 30 of drug delivery device 12 may still comprise the medication for a single dose or multiple doses. Cartridge 30 and needle assembly 50 may couple to housing 15 of drug delivery device 12 through any suitable coupling mechanism, including but not limited to threading, bayonet couplers, screws, rivets, snaps, ball-detent mechanisms, spring systems, pins, magnets, friction fitting, adhesives, or any other suitable coupling device, and combinations thereof. In embodiments where cartridge 30 and needle assembly 50 are separate components, any suitable coupling device such as those mentioned above may additionally be used to couple cartridge 30 and needle assembly 50. In the illustrated embodiment, second motor 57 is coupled to housing 15, and is not shown as part of needle assembly 50 or cartridge 30, but instead couples to needle assembly 50 when needle assembly 50 is coupled to housing 15. In other embodiments, second motor 57 may be part of needle assembly 50 and may be couplable and decouplable from housing 15 with needle assembly 50.

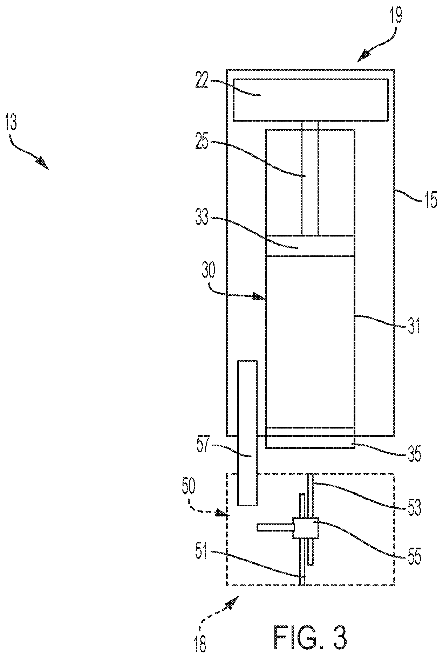

Referring now to FIG. 3, another embodiment of a drug delivery device 13 is shown. Drug delivery device 13 is similar to drug delivery device 11 of FIG. 1 and drug delivery device 12 of FIG. 2, except that cartridge 30 is fixedly coupled to housing 15, and needle assembly 50 is removably coupled to housing 15. Needle assembly 50 may be disposable while cartridge 30 and/or housing 15 may be reusable. In the illustrated embodiment, housing 15 and cartridge 30 may be used multiple times with different instances of needle assembly 50. In some embodiments, cartridge 30 may be refillable with the medication. As mentioned before with respect to drug delivery device 12, needle assembly 50 may be coupled to housing 15 through any suitable coupling device or system. Additionally, second motor 57 may be part of housing 15 or needle assembly 50. Additional embodiments, configurations, and details of drug delivery devices and their components will be discussed below.

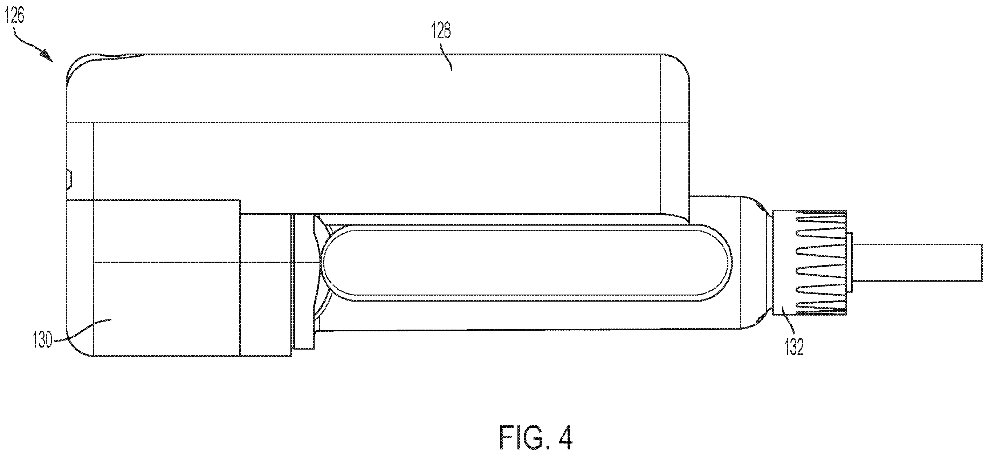

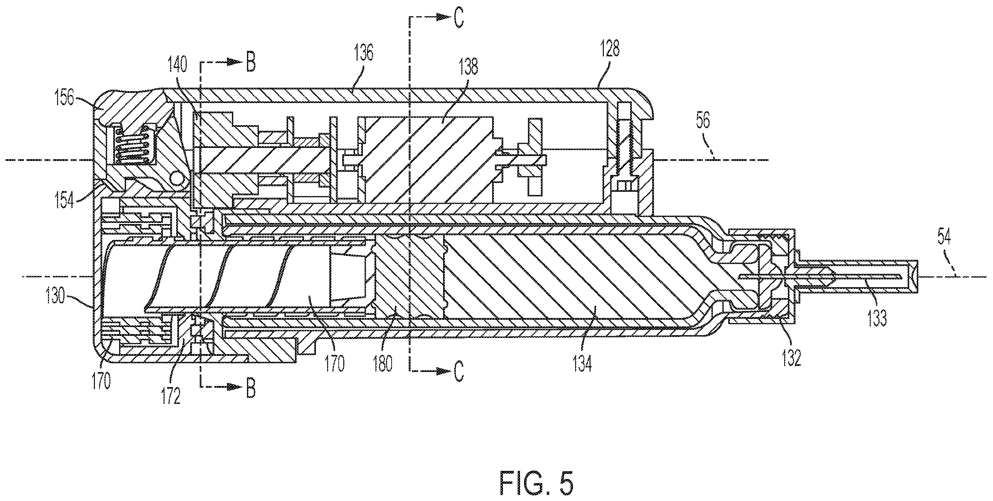

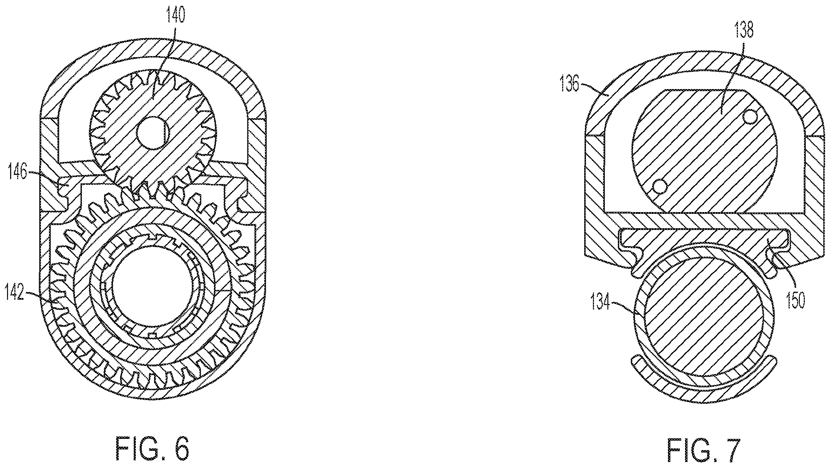

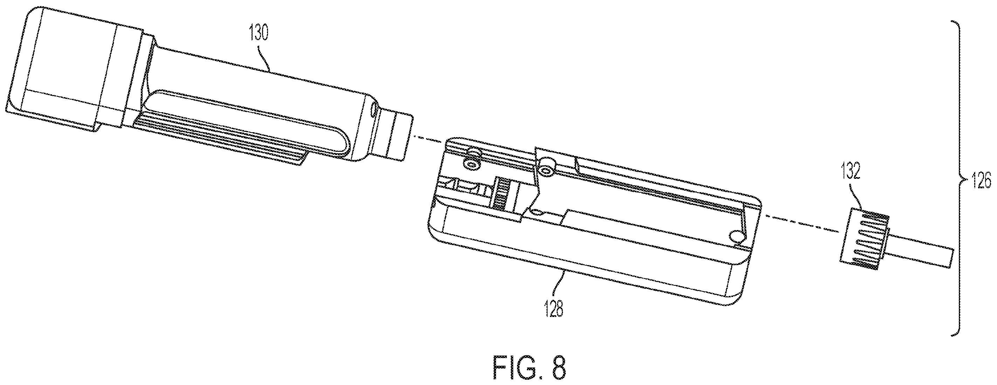

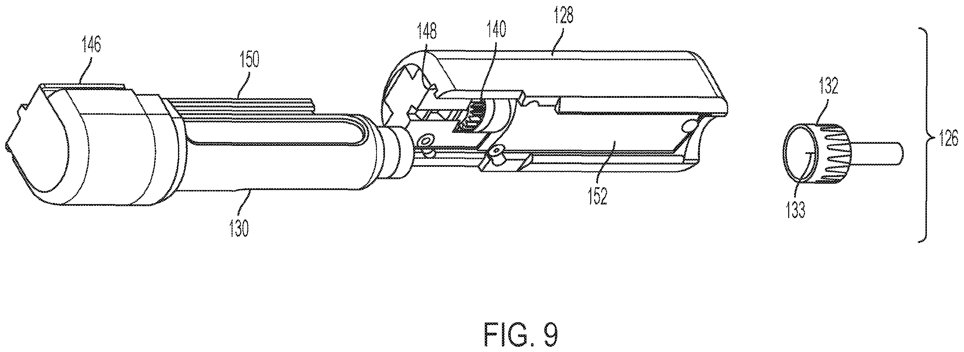

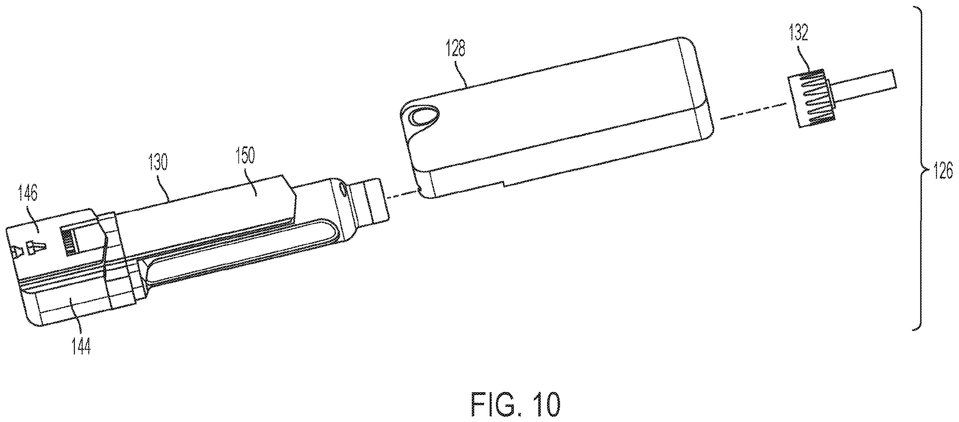

Referring to FIGS. 4-10, another embodiment of a drug delivery device 126 is provided. Drug delivery device 126 includes a reusable driving module 128 and a cassette 130. A needle assembly 132 is securable to the threaded distal end of a medication-containing cartridge 134. Reusable driving module 128 includes a housing 136 within which an electric motor 138 is mounted. A driving gear 140 is coupled to and driven by the output shaft of electric motor 138. A portion of driving gear 140 projects outwardly from housing 136 whereby it can engage a driven gear 142 within cassette 130 when cassette 130 is attached to reusable driving module 128.

Cassette 130 includes a housing 144 that defines a first T-shaped projection 146. Housing 136 defines a corresponding first T-shaped slot 148 which receives the first T-shaped projection 146. Cassette 130 also defines a second T-shaped projection 150 which is received by a second T-shaped slot 152 on housing 136. When the T-shaped projections 146, 150 are slid into the T-shaped slots 148, 152, a spring biased, pivoting latch member 154 on reusable driving module 128 engages a projecting lip on the cassette to prevent the cassette 130 from sliding out of engagement. Button 156 is depressed to disengage latch member 154. The drug delivery device 126 further comprises a drive ribbon 170 defining a first primary axis 54 and a drive member mechanism that defines a secondary parallel axis 56. The drive ribbon 170 is disposed within the cassette 130, and a trust member 172 controls the axial extension of drive ribbon 170.

The drug delivery device 126 may be used to administer the above-described medication to the patient through needle assembly 132. In the illustrated embodiment, the cassette 130 is configured to be decoupled from the reusable driving module 128. Generally, the drive ribbon 170 is configured to extend a needle 133 outward from needle assembly 132 along the first primary axis 54 and/or drive a drug through said needle 133 by pushing a stopper 180 through the cartridge 134. A number of examples of drug delivery device 126 and variations thereof are disclosed in PCT Publication No. WO 2019/112886, published on June 13, 2019, the entire disclosure of which is expressly incorporated by reference herein.

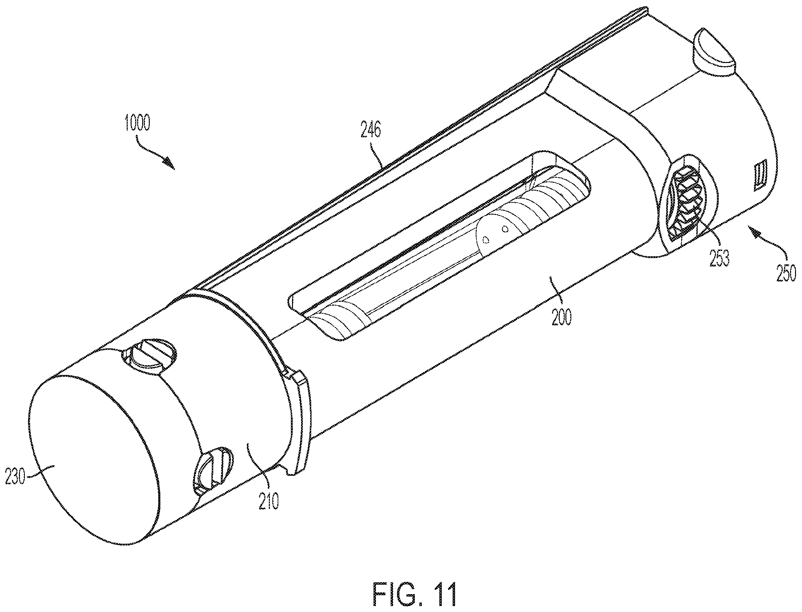

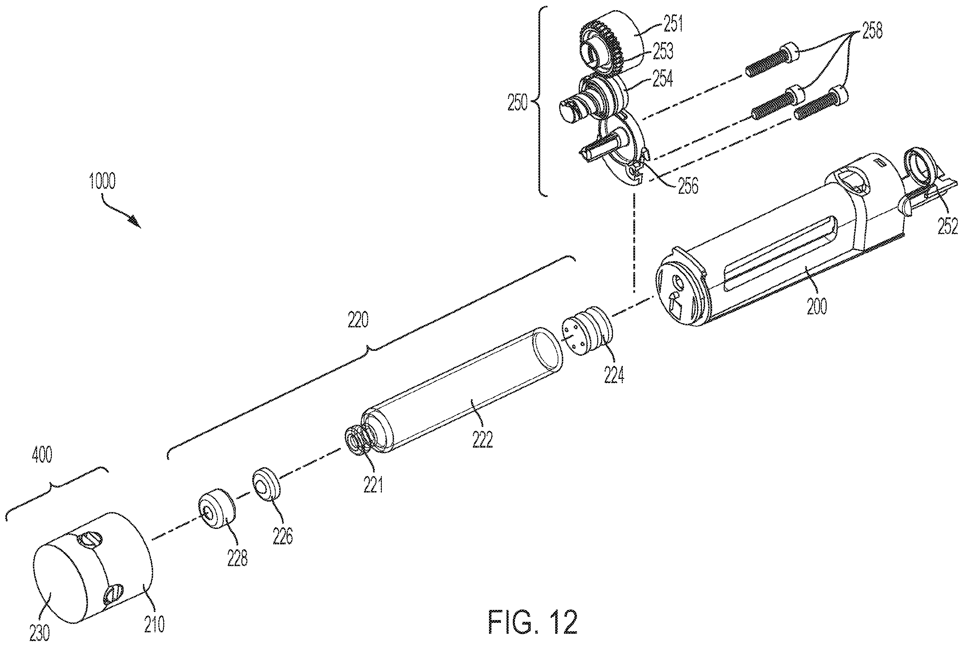

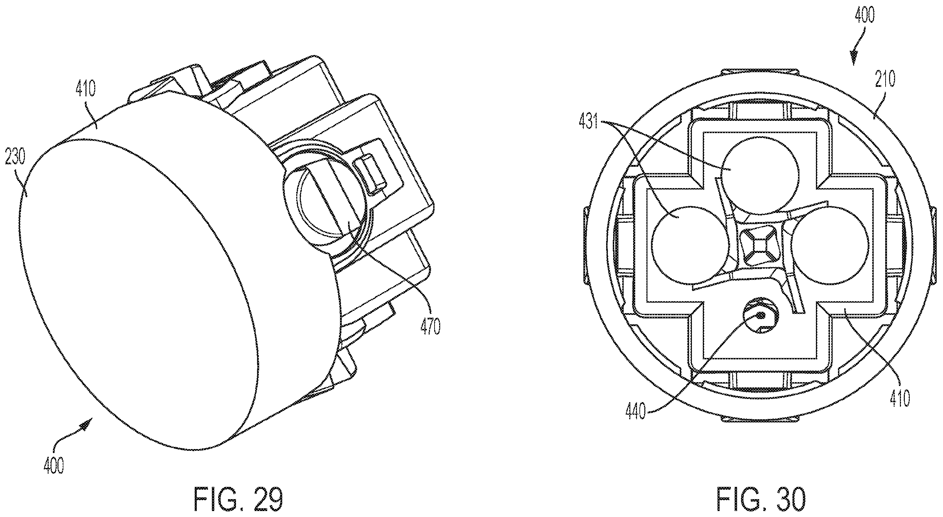

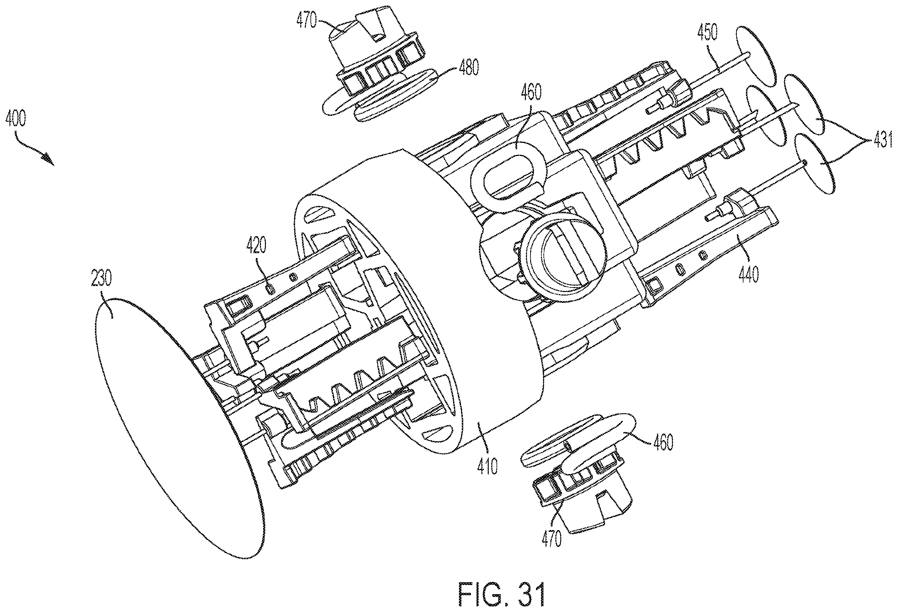

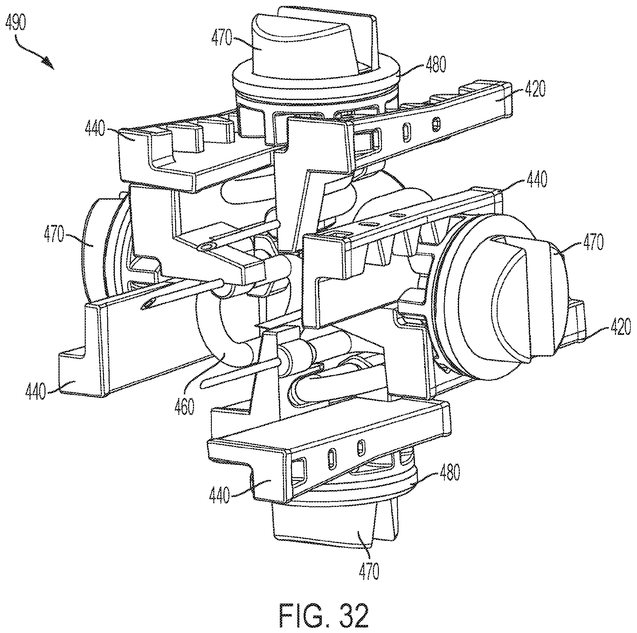

Referring now to FIGS. 11 and 12, another exemplary drug delivery device 1000 is shown. Drug delivery device 1000 comprises a cassette 200 configured for use with the driving module 128 of FIGS. 4-10 or another suitable driving module. The illustrative cassette 200 includes a driving system 250, a cartridge 220, and a needle assembly 400 (described further in FIGS. 29-32) having a seal 230. The illustrative cassette 200 also includes one or more mating elements such as T-shaped projections 246 (similar to T-shaped projections 146, 150) configured to interact with the corresponding driving module.

The cartridge 220 is positioned within cassette 200 and is configured to hold the above-described medication. The needle assembly 400 is generally configured to interact with a surface, for example a patient's skin, and to pierce a first needle (shown elsewhere) through the surface. Once the first needle has pierced the surface, the driving system 250 will drive the medication from within cartridge 220 through the needle assembly 400 and into or through the surface. Needle assembly 400 is then configured to retract the needle within the needle assembly 400. While the needle assembly 400 is shown in the illustrated embodiment of FIGS. 11 and 12, it should be understood that any embodiment of a needle assembly as disclosed herein may be used within drug delivery device 1000.

Like drug delivery device 126, in the illustrated embodiment the cassette 200 is configured to be coupled and decoupled from an element of drug delivery device 1000, in this case needle assembly 400. In the illustrated embodiment, needle assembly 400 is coupled to cassette 200 through a needle assembly coupler 210, but in other embodiments needle assembly 400 may be configured to couple directly to cassette 200 without the need for an additional coupler. Needle assembly 400 may be permanently or removably coupled to cassette 200 (as discussed above regarding FIGS. 1-3), and may couple to cassette 200 through any suitable coupling devices or systems, including but not limited to threading, bayonet couplers, screws, rivets, snaps, ball-detent mechanisms, spring systems, pins, magnets, friction fitting, adhesives, or any other suitable coupling device and combinations thereof. In other embodiments, needle assembly 400 may be an integral part of cassette 200. Needle assembly 400 may be a disposable component or may be used multiple times with different cassettes 200. Cassette 200 may be configured to be entirely or partially disposable. For example, cassette 200 and driving system 250 may be reusable, and cartridge 220 may be disposable. In such an embodiment, cartridge 220 may be couplable and decouplable from cassette 200 and/or driving system 250. In another embodiment, each of cassette 200, driving system 250, and cartridge 220 may be disposable, or the entirety of drug delivery device 1000 may be disposable.

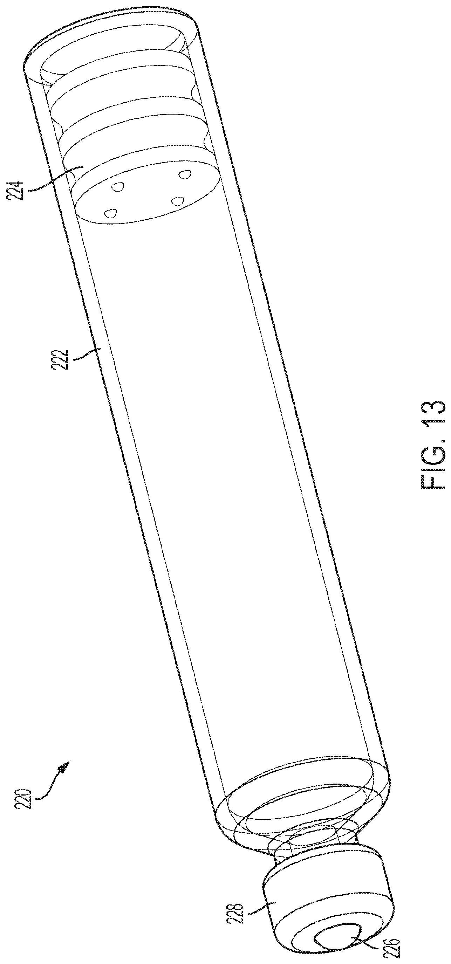

Referring now to FIGS. 12 and 13, cartridge 220 comprises a fluid housing 222, a stopper 224, a septum 226, and a septum seal 228. Fluid housing 222 is configured to hold a volume of the medication, which is retained within fluid housing 222 by the stopper 224 and the septum 226. The septum 226 is configured to be pierced by a septum needle (shown elsewhere) to allow for the fluid to flow through the needle that has pierced septum 226. The stopper 224 or piston is configured to slide along the interior of fluid housing 222 to increase the pressure within fluid housing 222 and drive the fluid through the septum needle within septum 226. In the illustrated embodiment, stopper 224 is driven by the driving system 250, but in other embodiments the stopper 224 may be driven by any mechanical, electrical, or human-activated drive system. Common drive systems include drive screws, linear drives activated, such as, for example, springs or electric motors, flexible rack/pinion drive, etc. The septum seal 228 is configured to retain septum 226 on a hub 221 of fluid housing 222. The cartridge 220 may be assembled and filled by any standard method as is known in the art. For example, septum 226 may be positioned against hub 221 and secured to hub 221 through septum seal 228. Fluid housing 222 may then be filled with a fluid and sealed with stopper 224. In an exemplary embodiment, each of the components of cartridge 220 is sterilized before use. Fluid housing 222 may be composed of glass, plastic, or any other material configured to retain a fluid. Septum seal 228, septum 226, and stopper 224 may be composed of an elastomer, plastic, or any other polymer or composite.

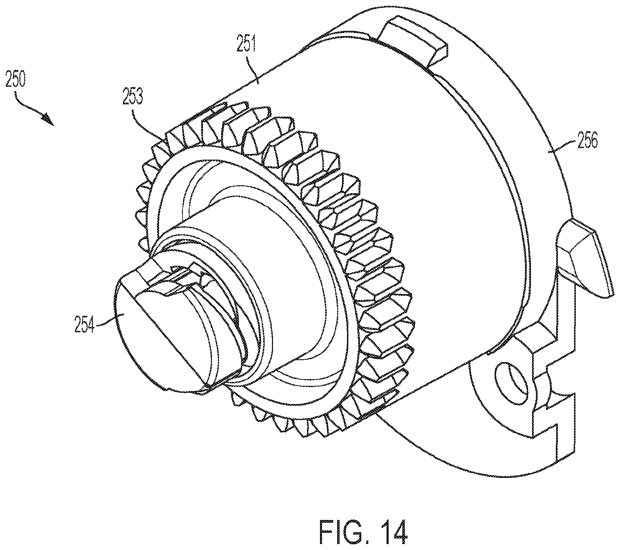

Referring now to FIGS. 12 and 14, an exemplary embodiment of the driving system 250 comprises a support 256, a collar 252, an activating member 251 having a driven gear 253, a driving member 254, and fasteners 258. The driven gear 253 of the activating member 251 is exposed through the cassette 200 and configured to be driven by a user or by an external mechanism (e.g. a motor such as first motor 22 shown in FIGS. 1-3) (similar to driven gear 142 of FIG. 4) to then engage driving member 254 and cause driving member 254 to drive stopper 224 within cartridge 220. In the illustrated embodiment, driving member 254 is a drive screw and is threaded to be driven by driven gear 253. Rotation of driven gear 253 causes axial movement of driving member 254 towards or away from stopper 224. In other embodiments, other driving systems may be used such as a rack/pinion driving system. The activating member 251 and driving member 254 are both supported by support 256, which is coupled to cassette 200 through collar 252 and fasteners 258. In the illustrated embodiment, fasteners 258 are screws, but in other embodiments may be any fastening device such as rivets, nails, clips, clamps, snaps, welds, adhesives, or other fastening devices or systems. Other exemplary embodiments of driving systems 250 are described in the PCT Publication No. WO 2019/112886, previously incorporated by reference.

Referring to FIGS. 14-16, first motor 22 is operably coupled to driven gear 253 such that activation of first motor 22 actuates driven gear 253. Additionally, drug delivery device 1000 optionally comprises a second motor 57 (as shown in FIGS. 1-3), configured to actuate a needle mechanism, such as any of the needle mechanisms 55, 390, 490, 590 described herein. In embodiments where drug delivery device does not comprise second motor 57, first motor 22 may be configured to actuate the needle mechanism(s).

Each of the first motor 22 and optional second motor 57 may be controlled by a motor controller 260. The term "logic" or "control logic" as used herein may include software and/or firmware executing on one or more programmable processors, application-specific integrated circuits (ASICs), field-programmable gate arrays (FPGAs), digital signal processors (DSPs), hardwired logic, or combinations thereof. Therefore, in accordance with the embodiments, various logic may be implemented in any appropriate fashion and would remain in accordance with the embodiments herein disclosed. Controller 260 may be included in drug delivery device 1000 or may be external. Controller 260 may include at least one processor 262 (e.g. microprocessor) that executes software and/or firmware stored in a memory 264 of controller 260. The software/firmware code contains instructions 265 that, when executed by the processor 262, cause controller 260 to perform the functions of the control algorithm described herein. Controller 260 may receive information from a plurality of system components and feed the information (e.g. medication data, patient data, drug delivery device data, needle assembly data) into the control algorithm which determines at least one drug delivery control parameter which may in part govern operation of first motor 22 and/or second motor 57. Controller 260 may include or be communicatively coupled to one or more interfaces to communicatively couple via one or more communication links to the drug delivery device 1000. Examples interfaces include wired and wireless signal transmitters and receivers. Example communication links include a wired communication link (e.g. a serial communication), a wireless communication link such as, for example, a short-range radio link, such as Bluetooth, IEEE 802.11, a proprietary wireless protocol, and/or the like. The term "communication link" may refer to an ability to communicate some type of information in at least one direction between at least two devices. The communication links may be a persistent communication link, an intermittent communication link, an ad-hoc communication link, and/or the like. Information may be transmitted via the communication links.

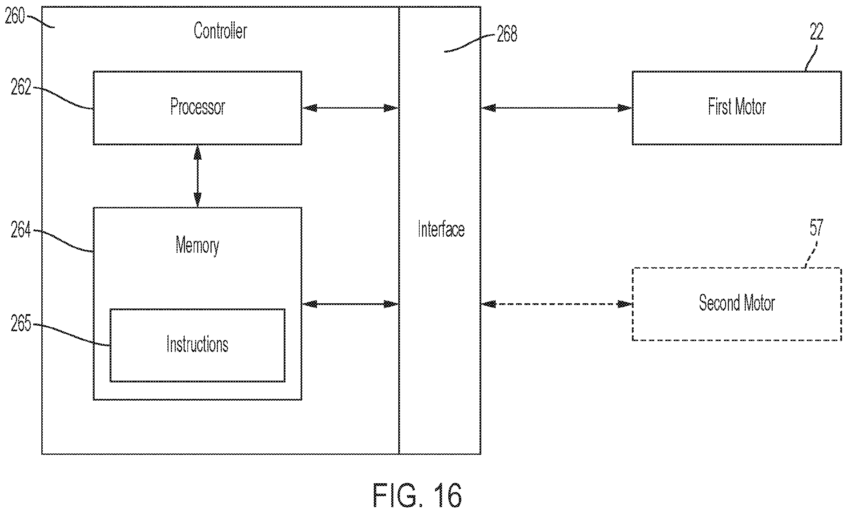

FIG. 16 shows the controller 260 including memory 264 and processor 262 communicatively coupled to the one or more interfaces 268 and to each other. The memory 264 may include computer-readable storage media in the form of volatile and/or nonvolatile memory and may be removable, non-removable or a combination thereof. In embodiments, memory 264 stores executable instructions 265 (e.g. computer code, machine-useable instructions, and the like) for causing processor 262 to implement aspects of embodiments of system components discussed herein and/or to perform aspects of embodiments of methods and procedures discussed herein, including the control logic described in more detail below. The memory 264, processor 262, and interfaces 268 may be communicatively coupled by one or more busses. The data and/or information may be provided to controller 260 as acquired, on a predefined schedule, or queued inn memory and supplied to controller 260 when requested.

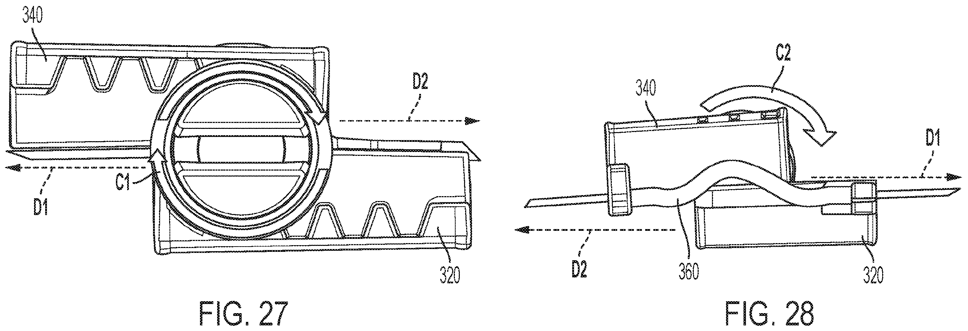

The logic of controller 260 may be configured to adjust the rate of actuation or the force of actuation provided by first motor 22 and/or second motor 57. The force of actuation by motors may be constant or variable. For example, if the medication in cartridge 220 is viscous, the force applied by first motor 22 to drive stopper 224 may be increased. In a further example, the rate of actuation for the first or second motor 22, 57 may be adjusted to alter the rate of actuation for the needle mechanism. The needle mechanism may be actuated more slowly to improve patient comfort and reduce irritation. In some embodiments, the controller may alter the operating parameters of first and/or second motor 22, 57 based on received information/data (e.g. patient data, medication data, historical use data, dose data, drug delivery device data, needle assembly data, cartridge data). Additionally, controller 260 may be configured to alter the speed of motors 22, 57 over the course of activation such that motion of driving member 254 and/or the needle mechanism is not constant or non-linear. For example, the speed/acceleration of the second motor 57 of needle mechanism may be controlled by a control algorithm in instructions 265 of controller 260 such that the needles decelerate near the end of travel of the needle supports to prevent a hard stop to minimize damage to components and/or cavitation of the medicament. Another advantage may be to control needle insertion speed into a patient to minimize pain, discomfort, or other undesirable sensations. The start angular position (where the needle mechanism is at a first, retracted configuration before use and/or after use, as shown in FIG. 27) and an end angular position (where the needle mechanism is at a second, extended configuration for use, as shown in FIG. 28) of a rotating actuator shaft, such as a drive shaft of the second motor, as well as incremental angular positions in between the start and end positions, which may be monitored via a position sensing assembly (not shown), including optical encoder, sliding contacts, magnetic sensing. Where the actuator shaft is within a percentage of travel within the start and/or end positions, such as, for example, at a predefined percentage in range of 1% to 5%, the rate of speed of second motor may be reduced such that the speed is zero or substantially reduced at the start/end angular positions. The rate of speed of second motor may have more than one setting for start and/or end positions, such as, for example, a high, medium, and low setting, which may be preconfigured at the manufacturing or provided to a HCP or patient via a user interface, such as a dial or button. Each of the settings may have a different percentage within end/start angular position and/or different rate of speed reduction, which can be tuned. The setting may be different between the start and end positions. For example, the rate during needle insertion may be different depending on the type/volume of medicament to provide a patient with as comfortable experience as can be had. Controller 260 may also control timing of motor activation, for example activating first motor 22 before second motor 57. Controller 260 may also receive data based on needle position indicating the location of at least one needle in the needle assembly, and may alter the speed, force, or positioning of the at least one needle based on the position data.

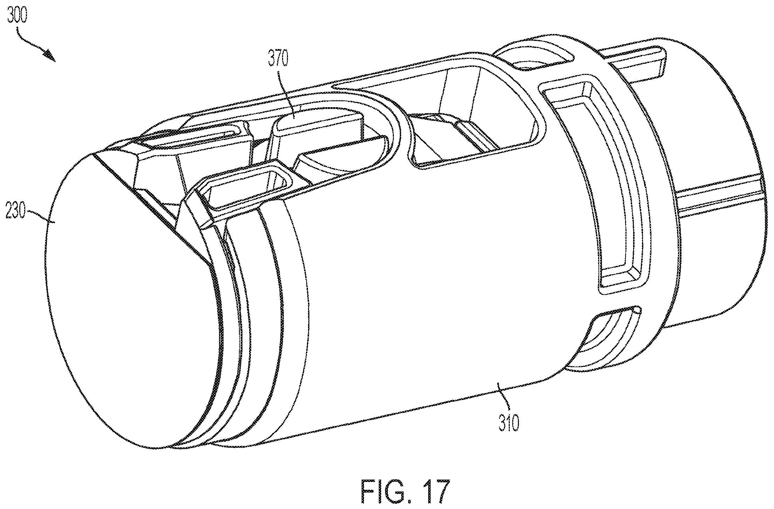

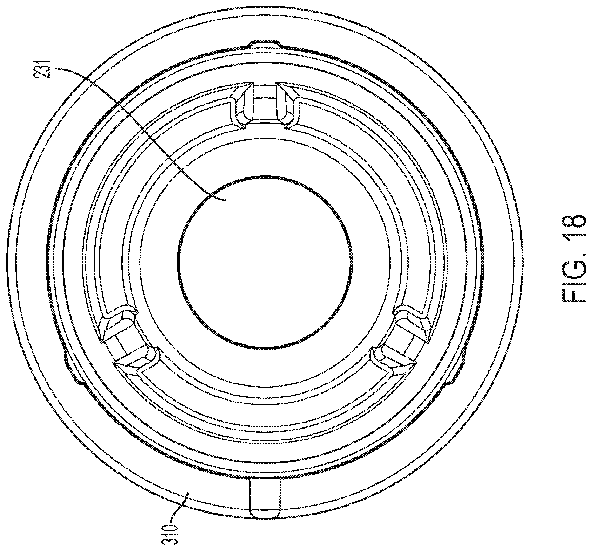

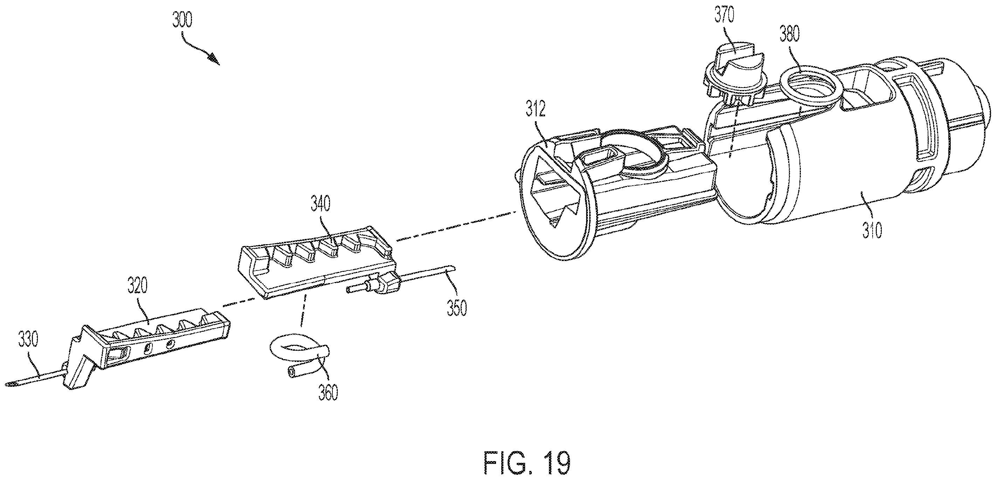

Referring now to FIGS. 17-20, an exemplary embodiment of a needle assembly 300 is shown. As discussed earlier, any of the illustrated needle assemblies or variations thereof may be used within drug delivery device 1000. Furthermore, the needle assemblies disclosed may be used separately from drug delivery device 1000, for example in chemical sampling instrumentation, or in other applications where a needle is used. Needle assembly 300 comprises an assembly housing 310, an internal housing 312, a first needle support 320 coupled to a first needle 330, a second needle support 340 coupled to a second needle 350, a movable connector 360, a driving member 370, and an O-ring 380. Furthermore, needle assembly 300 comprises an exterior seal 230 and an interior seal 231. Together, first needle support 320, first needle 330, second needle support 340, second needle 350, movable connector 360, and movable connector 360 comprise a needle mechanism 390. The exterior seal 230 and interior seal 231 are configured to seal the needle mechanism 390 within the needle assembly 300 before the needle assembly 300 is used and also to help maintain sterile conditions within needle assembly 300.

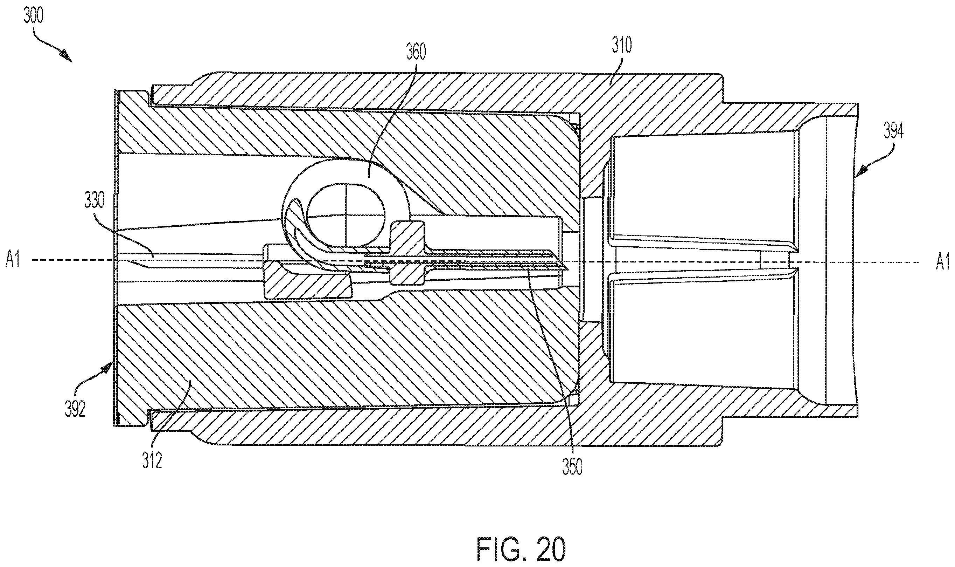

In the illustrated embodiment, needle assembly 300 is configured to interact with the patient's skin for delivery of the above-described medication. A first or distal end 392 of needle assembly 300 is positioned against the skin, and a second or proximal end 394 of needle assembly 300 interacts with cassette 200. The needle assembly 300 is configured to couple to cassette 200 either directly, or through needle assembly coupler 210. The first end 392 and/or the exterior seal 230 may comprise an adhesive to assist in positioning the needle assembly 300 against the patient's skin and may also comprise fasteners or adhesives to secure the needle assembly 300 to cassette 200. As shown in FIG. 20, the internal housing 312 is supported by the housing 310, and the needle mechanism 390 is supported by the internal housing 312. In other embodiments, needle assembly 300 may comprise only one housing, and internal housing 312 may be integral to assembly housing 310.

Referring to FIGS. 20-24, the needle mechanism 390 is configured to fit entirely within needle assembly 300 when in a first configuration. FIGS. 20-24 show various views of needle mechanism 390 in the first configuration. In the first configuration, first needle 330 and second needle 350 are approximately aligned parallel to one another in the direction of central axis A1. As shown, first needle 330 and second needle 350 are pointed in approximately opposite directions along axis A1. First needle 330 is pointed towards first end 392 of needle assembly 300, and second needle 350 is pointed towards second end 394 of needle assembly 300. As will be described later, first needle 330 and second needle 350 are configured to move generally along axis A1 when the needle assembly 300 is activated.

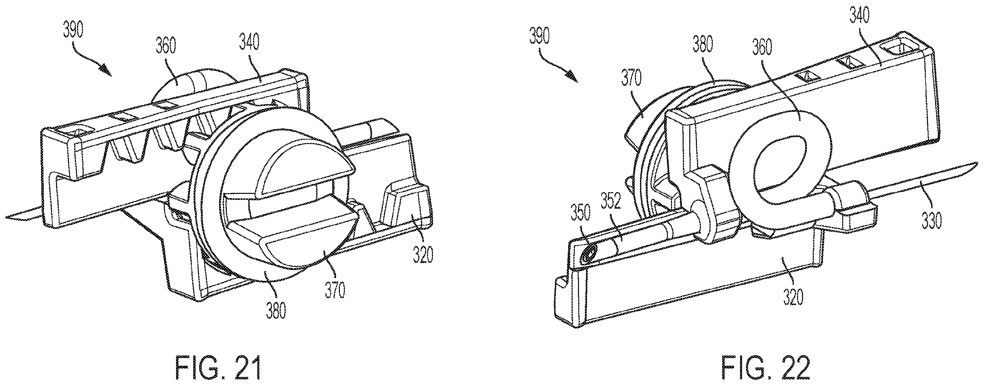

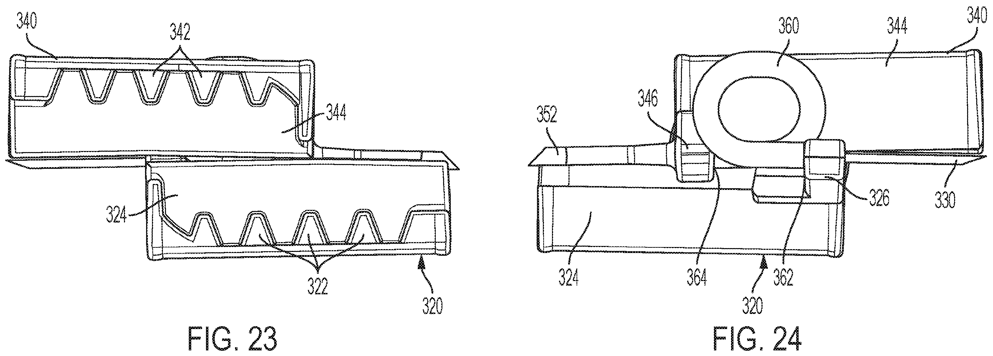

Referring to FIGS. 21-28, an exemplary embodiment of needle mechanism 390 is shown. First needle support 320 and second needle support 340 are positioned generally along a common plane and are slidably coupled to one another through driving member 370. A first side of first and second needle supports 320, 340 is configured to interact with driving member 370, and a second side of first and second needle supports 320, 340 is configured to interact with and support first and second needles 330, 350. In the illustrated embodiment, first and second needle supports 320, 340 are generally rectangular in shape and are configured to fit within needle assembly 300, as shown in FIG. 20. In other embodiments, first and second needle supports 320, 340 may be any shape to accommodate motion of needles 330, 350 and fit within needle assembly 300. In the illustrated embodiment, needle mechanism 390 comprises a rack and pinion configuration, wherein each of first needle support 320 and second needle support 340 is a rack and driving member 370 includes a pinion gear. First needle support 320 comprises a support body 324, support engagement features 322, and a needle retainer 326. The needle retainer 326 is configured to couple with first needle 330 by inserting at least of portion of first needle 330 into needle retainer 326, thereby coupling first needle 330 to first needle support 320. Similarly, second needle support 340 comprises a support body 344, support engagement features 342, and a needle retainer 346, and the second needle 350 is coupled to second needle support 340 through needle retainer 346. As shown best in FIGS. 22 and 24, one or both of first needle support 320 and second needle support 340 may additionally comprise a needle guard 352, which may function to protect the needle and/or to provide additional strength to the needle. The illustrative needle guard 352 is a cylindrical housing with a bore extending therein, through which the respective needle 330, 350 extends.

First needle 330 and second needle 350 are fluidly coupled to one another through movable connector 360. Movable connector 360 is configured to be movable relative to each of the needles 330 and 350. Movable connector 360 may be movable by flexing, bending, stretching, or otherwise deforming. Accordingly, movable connector 360 may be composed of a flexible material, such as an elastomer, thermoset polymer, or rubber. In an exemplary embodiment, movable connector 360 is composed of silicone. In another embodiment, movable connector 360 may be composed of rigid materials coupled together through flexible connections. In yet another embodiment, movable connector 360 may be formed of relatively rigid materials and may move through a telescoping action. In the illustrated embodiment of FIG. 24, movable connector 360 is a flexible tube having a first end 362 fluidly coupled to the end of the first needle 330 and a second end 364 fluidly coupled to the end of the second needle 350. The ends 362, 364 of the flexible tube of the movable connector 360 may be coupled directly to the needle ends 330, 350 or to the respective needle retainers 326, 346. As described further below, the ends 362, 364 of the flexible tube are configured to move with the needles 330, 350.

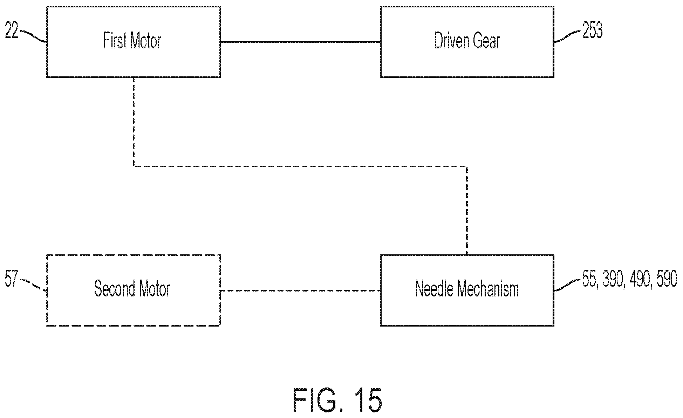

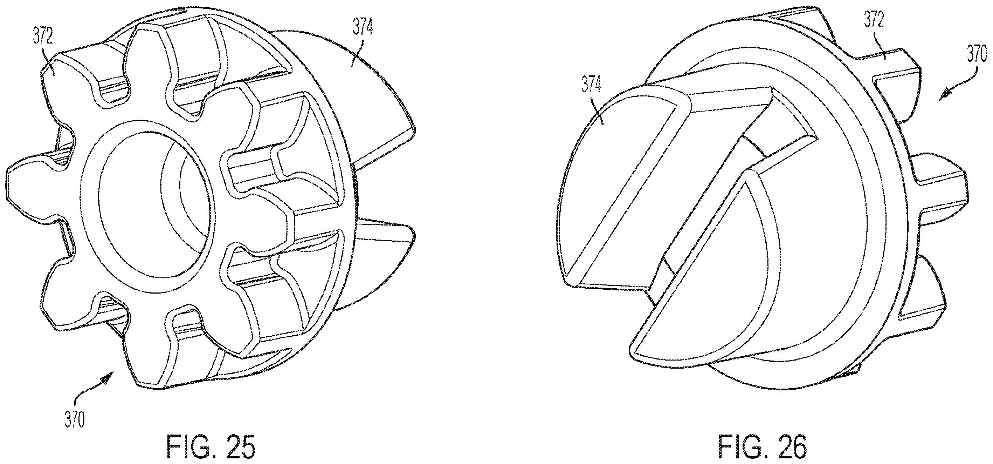

As shown best in FIGS. 25 and 26, driving member 370 comprises driving engagement features 372, and an actuating member 374. The O-ring 380 may be provided on driving member 370 in order to provide a better seal between driving member 370 and assembly housing 310, or to allow driving member 370 to rotate more easily within assembly housing 310. The driving engagement features 372 are configured to interact with the support engagement features 322 and 342 (FIG. 23). In the illustrated example, driving engagement features 372 and support engagement features 322 and 342 are teeth or protrusions configured to mesh together in a rack and pinion mechanism. In other embodiments, any of the engagement features within needle mechanism 390 may be other features configured to interact with one another such as protrusions and recessions, screws and threads, zipper type features, or any other complimentary engagement features. Furthermore, in the illustrated example, actuating member 374 is configured to be actuated by a rotation of a flat object, such as a flat-head screwdriver, of an actuator shaft (not shown). In other embodiments, actuating member 374 may be actuated by a shaft of an electric motor, a shaft connected to a torsional spring, or human interaction such as rotation of a knob. In one exemplary embodiment, both the driving system 250 and driving member 370 are each independently driven by an electric motor, such as first motor 22 and second motor 57 (FIG. 15). One electric motor may control the movement of both driving system 250 and driving member 370, or they may each have their own respective actuating motors.

Referring now to FIGS. 27-28, the needle mechanism 390 is configured to allow for movement of first needle support 320 and second needle support 340 upon activation of driving member 370. Needle mechanism 390 is movable between a first, retracted configuration before use and/or after use, as shown in FIG. 27, and a second, extended configuration during use, as shown in FIG. 28.

In the first, retracted configuration of FIG. 27, both first needle 330 and second needle 350 are axially positioned entirely within assembly housing 310. Movable connector 360 may be arranged in a compressed (e.g., bent or looped) state between closely-positioned tube ends 362, 364 and needles 330, 350 in the first configuration, where the closely-positioned tube ends 362, 364 are separated by a first distance. Upon rotation of driving member 370 in a direction C1, first needle support 320 and first needle 330 are driven in a direction D1, and second needle support 340 and second needle 350 are driven in a direction D2 until the rotation of driving member 370 stops once the needle mechanism 390 is in the second configuration. In the illustrated embodiment, D1 and D2 are approximately parallel within a plane and are generally opposite of one another. Furthermore, they are approximately parallel to axis A1 (See FIG. 20). In other embodiments, D1 and D2 may be angled relative to one another, and may not be approximately parallel to A1.

In the second, extended configuration, first needle 330 extends axially beyond assembly housing 310 and passes through the outer seal 230 and into whatever surface needle assembly 300 is positioned against. Additionally, second needle 350 extends axially beyond assembly housing 310 and passes through interior seal 231 and septum 226 into fluid housing 222. Because the movable connector 360 is movable, it maintains fluid communication between first needle 330 and second needle 350 throughout movement of the needle mechanism 390. Movable connector 360 may be arranged in an extended state between the now-distant tube ends 362, 364 and needles 330, 350 in the second configuration, where the now-distant tube ends 362, 364 are separated by a second distance larger than the first distance of the first configuration. In the second configuration, first needle 330 is fluidly coupled to cartridge 220, so the fluid within fluid housing 222 is capable of flowing through second needle 350, movable connector 360, and first needle 330 into whatever body or surface first needle 330 has pierced. In an exemplary embodiment, the driving system 250 is activated after needle mechanism 390 is in the second configuration, and the fluid within cartridge 220 is driven out and into the body or surface pierced by first needle 330. In another embodiment, a number of components of needle mechanism 390, and/or the internal housing 312 comprise a stopping feature to physically stop the movement of first needle support 320 and second needle support 340 once needle mechanism 390 reaches the second configuration. Such a stopping feature may be a blocking member, a protrusion, a detent, or an absence of engagement members within a portion of the needle mechanism 390.

From the second configuration, needle mechanism 390 is configured to be movable back to the first configuration through reverse activation of driving member 370 as well. As shown in FIG. 28, driving member 370 may be rotated in a direction C2, which is generally opposite to C1, in order to drive first needle support 320 in the direction D2, and second needle support 340 in the direction D1. In an exemplary embodiment, needle mechanism 390 is moved from the second configuration to the first configuration after the fluid has been driven out of cartridge 220 and through needle mechanism 390. In an embodiment where drug delivery device 1000 is being used to deliver the above-described medication to the patient, extending the first needle 330 from needle assembly 300, and then retracting the first needle 330 back into the needle assembly 300 after injection would allow the patient to be injected with a medication without needing to directly observe or handle the first needle 330.

Referring now to FIGS. 29-32, needle assembly 400 is shown. Needle assembly 400 is similar to needle assembly 300, but instead of a single needle mechanism, needle assembly 400 comprises a plurality of needle mechanisms 490. In the illustrated embodiment, four needle mechanisms 490 are shown, but any number of needle mechanisms 490 may be used. Each of the needle mechanisms 490 comprise a first needle support 420 coupled to a first needle 430, a second needle support 440 coupled to a second needle 450, a movable connector 460, a driving member 470, and an O-ring 480. Each needle mechanism 490 within needle assembly 400 operates essentially the same as the individual needle mechanism 390 within needle assembly 300, as described above. In an exemplary embodiment, only one of the needle mechanisms 490 is activated at a time, and so only one of the needle mechanisms 490 is in the second, extended configuration when needle assembly 400 is used. Furthermore, in the exemplary embodiment, each of the needle mechanisms 490 are sterile before use.

Needle assembly 400 additionally comprises a housing 410, and a plurality of interior seals 431. The interior seals 431 are configured to function similarly to interior seal 231, but accomplished with a plurality of discrete, smaller seals instead of one larger seal. As shown best in FIG. 30, the interior seals 431 (one of which is omitted in the illustrated embodiment) are positioned to cover holes in housing 410. When the cassette 200 is coupled to needle assembly 400, the cassette 200 may comprise a single cartridge 220 to align with a single interior seal 431 or may comprise a number of cartridges 220 to align with each of the interior seals 431.

Needle assembly 400 may also comprise a rotating assembly (not shown). The rotating assembly may rotate the entirety of needle assembly 400, or just the needle mechanisms 490 within needle assembly 400 in order to align one of the needle mechanisms 490 with the cartridge 220. The needle mechanism 490 that is aligned with the cartridge 220 may then move from the first configuration to the second configuration (as described with respect to needle mechanism 390) to cause the second needle support 440 to pierce the interior seal 431 and septum 226, and first needle support 420 to pierce seal 230 and the surface or skin that needle assembly 400 is positioned against. The needle mechanism 490 may then be moved back to the second configuration (e.g. after medication delivery as described with respect to needle assembly 300), and then the rotating assembly can rotate another needle mechanism 490 to align with cartridge 220. The new needle mechanism 490 to be aligned with cartridge 220 may then be activated to pierce the same cartridge 220 again, or a new cartridge 220 may be used. In an exemplary embodiment, the rotating assembly is a gear to be driven by another gear, a screw, a rack, or another gear engaging feature. Additionally, the rotating assembly may be driven by an electric motor.

In another embodiment, at least one of the rotating assembly, the housing 410, and the needle mechanisms 490 have an engagement feature to stop the rotation assembly in the correctly aligned configuration. Examples of engagement features include indents, grooves, latches, protrusions, detent-and-ball mechanisms, and other locking features. Having multiple needle mechanisms 490 within needle assembly 400 allows for multiple doses of a medication to be delivered with the same needle assembly 400, such that a different, sterile needle is used for each injection. Additionally, different medications may be injected in through the same needle assembly 400, but through different needle mechanisms 490. In some embodiments, drug delivery device 1000 comprises a counter or a needle counter (not shown) that tracks how many and/or which needles have been used. For example, a needle index may increase after a needle mechanism 490 has been extended from the first configuration to the second configuration, or when the needle assembly 400 rotates. Drug delivery device 1000 may indicate to a user when each of the available needles has been used.

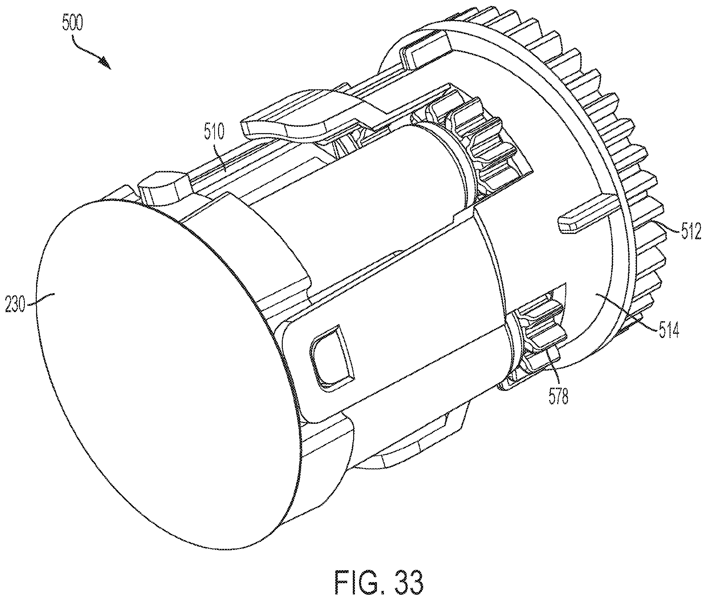

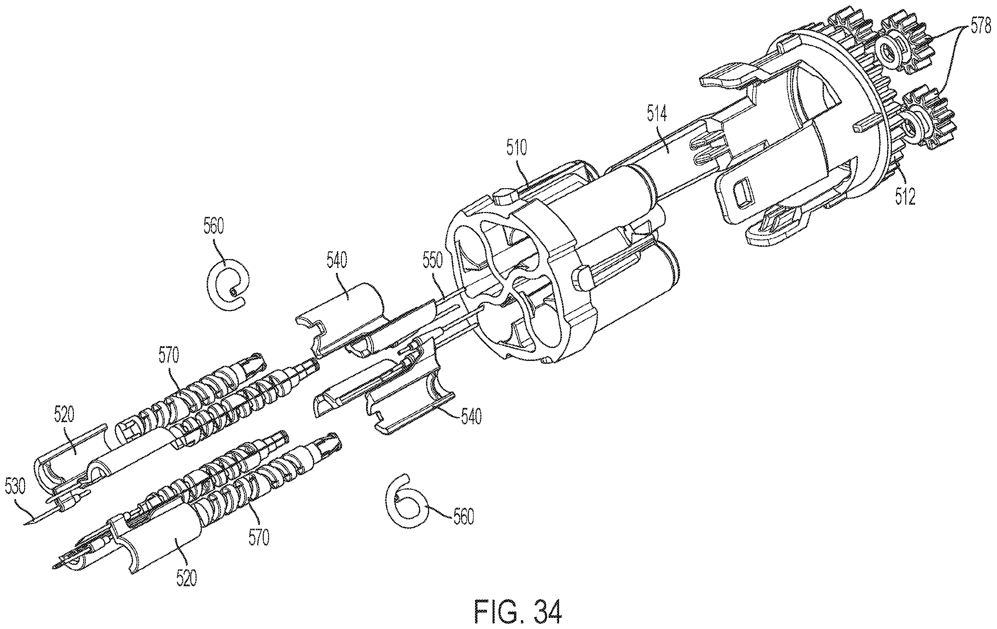

Referring now to FIGS. 33-34, another embodiment of a needle assembly 500 is shown. Needle assembly 500 comprises a housing 510, a rotating assembly 514, and a plurality of needle mechanisms 590. Each needle mechanism 590 comprises a first needle support 520 coupled to a first needle 530, a second needle support 540 coupled to a second needle 550, a movable connector 560, a driving member 570, a driving member actuator 578, and an O-ring 580. In the illustrated embodiments, a plurality of needle mechanisms 590 are shown, but any number of needle mechanisms 590 may be used, including a single needle mechanism 590, similar to the embodiment shown in FIG. 17. Movable connector 560 may have various features in common with the above-described movable connector 360.

Rotating assembly 514 comprises an exposed driven gear 512 and is configured to couple with housing 510. In an exemplary embodiment, driven gear 512 is driven by a motor to rotate housing 510 through rotating assembly 514. In other embodiments, rotating assembly 514 may not comprise driven gear 512, and may instead have a different feature to facilitate its rotation, such as a grip to be rotated by a user, or other engagement features to be rotated by a motor or another device. In still other embodiments, rotating assembly 514 may be configured to only rotate the needle mechanisms 590 within housing 510 instead of rotating the entire needle assembly 500.

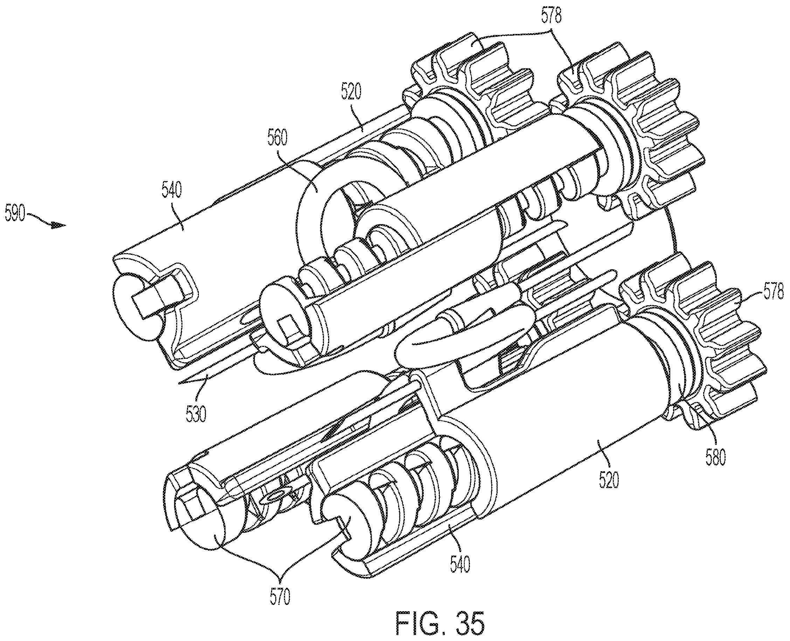

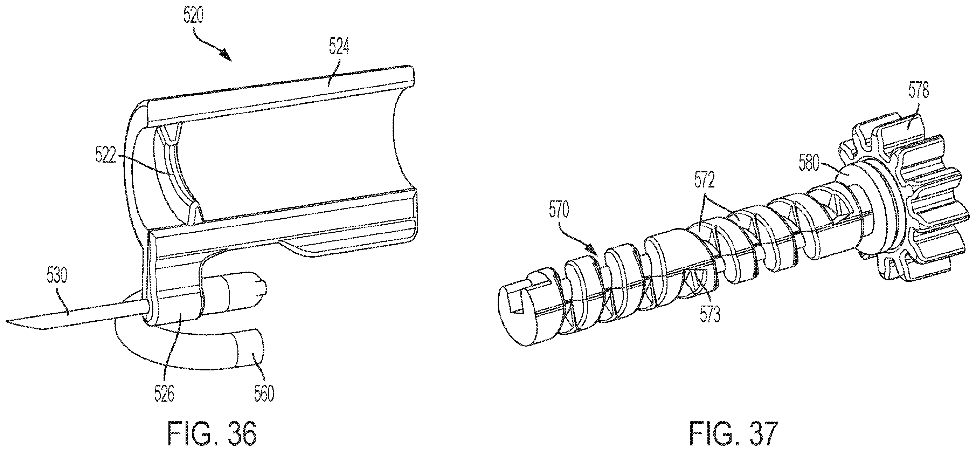

Referring now to FIGS. 35-39, the needle mechanisms 590 of needle assembly 500 are configured as a drive screw assembly. Each of the first needle support 520 and the second needle support 540 comprise an engagement feature 522, a support body 524, and a needle retainer 526. The first needle support 520 and second needle support 540 are configured to engage with driving member 570. Each of first and second needle supports 520, 540 is generally curved to accommodate interaction with driving member 570 and is configured to position first and second needles 530, 550 generally proximate one another. In other embodiments, first and second needle supports 520, 540 may be configured to position first and second needles 530, 550 on opposite sides of a central axis from one another. Each engagement feature 522 is configured to interact with a driving engagement feature 572 of driving member 570. In the illustrated embodiments, engagement feature 522 is in threaded engagement with the driving member 570. Engagement feature 522 is shown as a thread, driving member 570 is shown as a threaded shaft such as a screw, and driving engagement feature 572 is a trough or root within the screw. The engagement feature 522 is configured to be received within or otherwise interact with the driving engagement feature 572 of driving member 570. In the illustrated embodiment, driving engagement features 572 of driving member 570 are oppositely oriented along opposing halves of driving member 570 such that rotation of driving member 570 causes opposite axial movement of first and second needle supports 520, 540. A driving gear 578 is coupled to each driving member 570 such that rotation of driving member actuator 578 causes rotation of driving member 570. Driving member 570 also comprises at least one stopping feature 573 configured to stop a movement of first needle support 520 and/or second needle support 540 along driving member 570. In the illustrated embodiment, driving member 570 comprises a stopping feature 573 on each half of driving member 570.

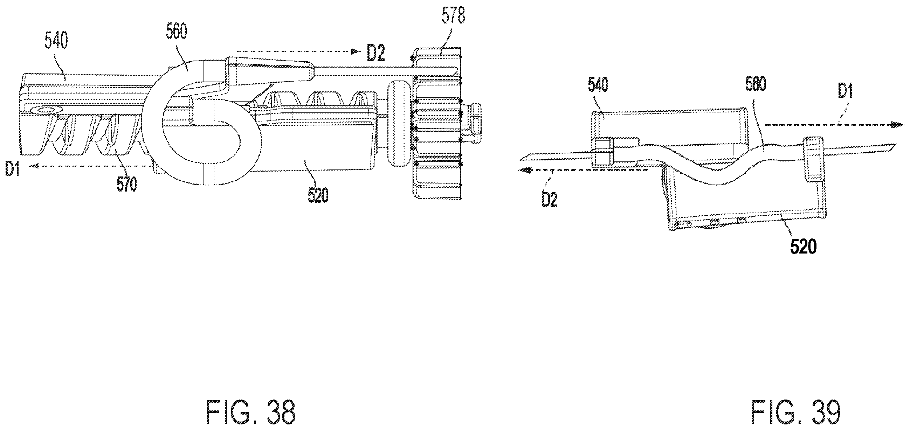

Referring now to FIGS. 38-39, the needle mechanisms 590 may be extended and retracted similarly to the needle mechanisms 390 and 490, but through the use of a different driving mechanism. More specifically, the needle mechanisms 590 may be moved between a first, retracted configuration before use and/or after use, as shown in FIG. 38, and a second, extended configuration during use, as shown in FIG. 39. Within a needle mechanism 590 of needle assembly 500, the driving member actuator 578 is rotatable locked with the driving member 570, and thus when rotated, the rotation of driving member 570 is caused. The engagement feature 522 on first needle support 520 and second needle support 540 are configured to move first needle support 520 and second needle support 540 in opposite axial directions along driving member 570 when driving member 570 is rotated. Each needle mechanism 590 begins in the first configuration, as shown in FIG. 38, and rotation of driving member 570 in a first direction causes first needle support 520 to move in the direction D1, and second needle support 540 to move in the direction D2, thereby moving the needle mechanism 590 into the second configuration. The driving of member actuator 578 stops the movement of first needle support 520 and second needle support 540 once the needle mechanism 590 reaches the second configuration. The driving member 570 may then rotate in a second, opposite direction to move the needle mechanism 590 from the second configuration to the first configuration by moving first needle support 520 in the direction D2, and second needle support 540 in the direction D1. All previous disclosure related to the first configuration, second configuration, needles, uses, etc. as discussed regarding needle assembly 300 may be applied to needle assembly 500. Furthermore, all previous disclosure related to multiple needle mechanisms and rotating assemblies as discussed regarding needle assembly 400 may also be applied to needle assembly 500. Utilizing a screw mechanism as in needle assembly 500 may allow needle assembly 500 to occupy less space than rack and pinion mechanisms as in needle assembly 300 and needle assembly 400.

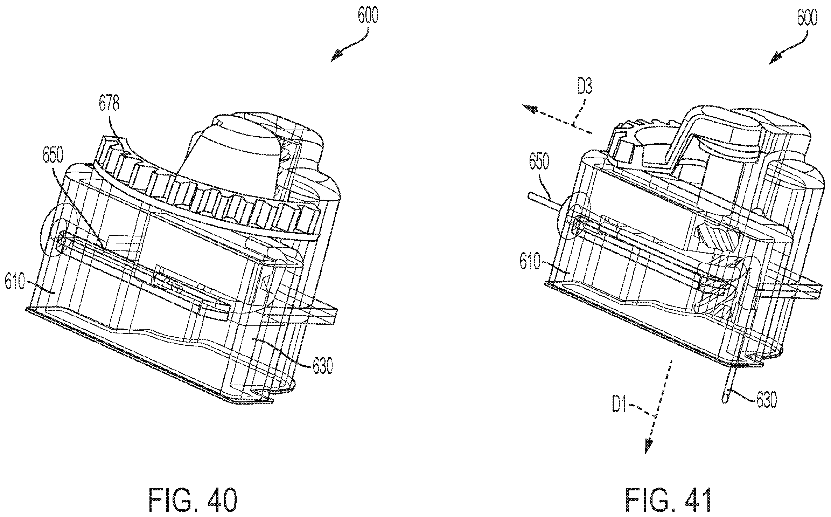

Referring to FIGS. 40-42, another embodiment of a needle assembly 600 is shown. Needle assembly 600 functions similarly to previously described embodiments of needle assemblies 300, 400, 500, except that needle assembly 600 generates non-parallel needle movement. Needle assembly 600 comprises a housing 610, first needle 630 supported by a first needle support 620, a second needle 650 supported by a second needle support 640, a movable connector 660 configured to fluidly couple first and second needles 630, 650, a driving member 670 configured to cause movement of first and second needle supports 620, 640, an O-ring 680, and a driven gear member 678 configured to be driven by an external source (e.g. a motor) to in turn drive the driving member 670. Additionally, needle assembly 600 comprises a first seal 230 and at least one second seal 631.

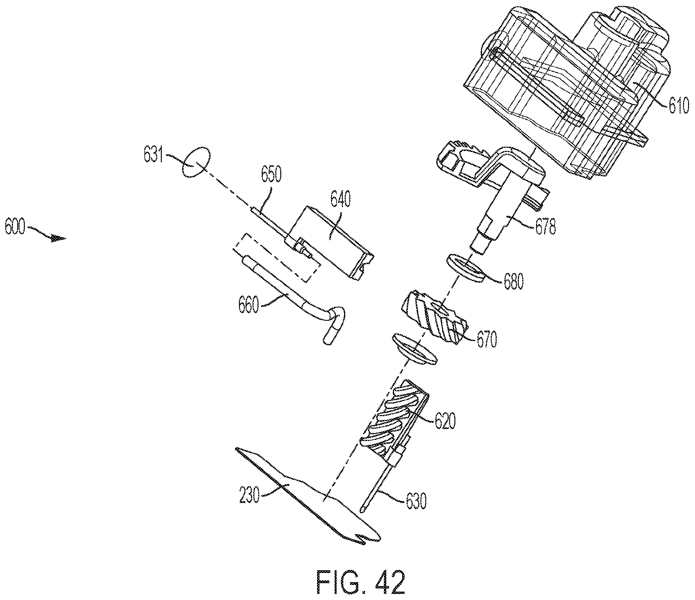

Like the previously-described embodiments of needle assemblies 300, 400, 500, the needle assembly 600 may be moved between a first, retracted configuration before use and/or after use, as shown in FIG. 40, and a second, extended configuration during use, as shown in FIG. 41. In the first configuration of FIG. 40, first needle 630 and second needle 650 are both retracted into housing 610. In the second configuration of FIG. 41, first needle 630 is extended from housing 610 in a first direction D1, and second needle 650 is extended from housing 610 in a second direction D3, wherein D1 is generally not parallel to D3. In some embodiments, D1 is generally orthogonal to D3. As shown best in FIG. 42, the driving member 670 is configured as a pinion, and the first and second needle supports 620, 640 are generally configured as threaded racks configured to engage opposing sides of the driving member 670. In this arrangement, rotation of driving member 670 causes axial movement of first and second needle supports 620, 640, in generally non-parallel directions D1, D3.

Multiple embodiments of needle assemblies and needle mechanisms have been disclosed. It should be understood that the features and configurations of each embodiment disclosed may be applied to any other embodiment disclosed, either alone or in combination with other features and configurations. Many of the features discussed are attributed to drug delivery device 1000, but they may also be applied to any other embodiment of drug delivery device 11, 12, 13, 126 described herein, or any modifications thereof.

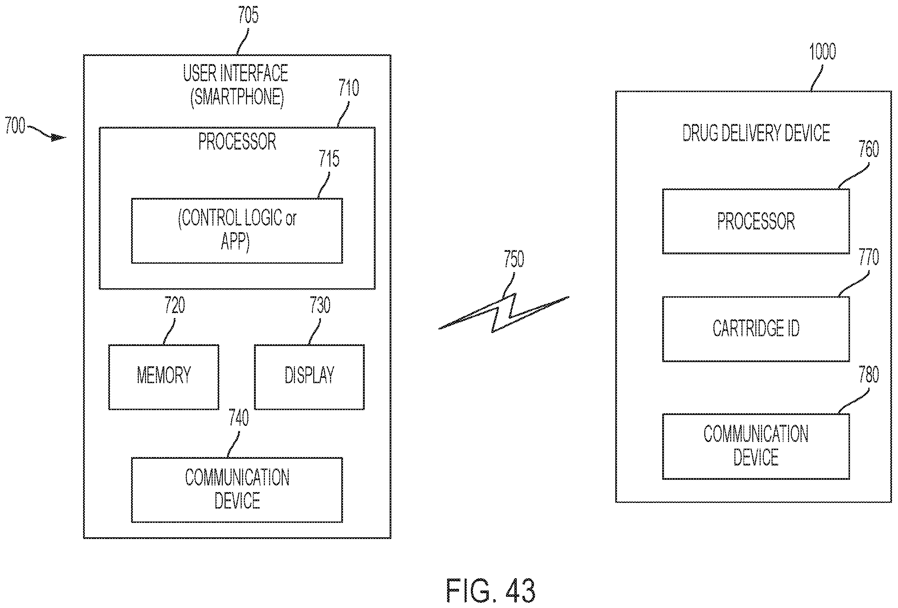

Referring now to FIG. 43, a computing device 700 may be used to communicate with and/or operate any of the drug delivery devices 11, 12, 13, 126, 1000 described herein, illustratively drug delivery device 1000. Computing device 700 illustratively includes a mobile device, such as a smartphone. Alternatively, any suitable computing device may be used, including but not limited to a laptop, desktop, tablet, or server computer, for example.

The computing device 700 includes at least one processor 710 that executes software and/or firmware stored in memory 720 of device 700. The software/firmware code contains instructions that, when executed by processor 710, causes device 700 to perform the functions described herein. The at least one processor 710 illustratively includes control logic and/or an application 715 operative to activate drug delivery device 1000. Memory 720 is any suitable computer readable medium that is accessible by processor 710. Memory 720 may be a single storage device or multiple storage devices, may be located internally or externally to processor 710, and may include both volatile and non-volatile media. Exemplary memory 720 includes random-access memory (RAM), read-only memory (ROM), electrically erasable programmable ROM (EEPROM), flash memory, a magnetic storage device, optical disk storage, or any other suitable medium which is configured to store data and which is accessible by processor 710.

Computing device 700 includes a user interface 705 in communication with processor 710 and operative to provide user input data to the system and to receive and display data, information, and prompts generated by the system. User interface 705 includes at least one input device for receiving user input and providing the user input to the system. In the illustrated embodiment, user interface 705 is a graphical user interface (GUI) including a touchscreen display operative to display data and receive user inputs. The touchscreen display allows the user to interact with presented information, menus, buttons, and other data to receive information from the system and to provide user input into the system. Alternatively, a keyboard, keypad, microphone, mouse pointer, or other suitable user input device may be provided.

Computing device communicates with drug delivery device 1000 through signal 750. Signal 750 may be a wireless or wired signal. Drug delivery device 1000 may comprise a processor 760 similar to processor 710, a cartridge ID 770, and/or a communication device 780. Communication device 780 may send or receive a signal 750 to/from communication device 740, or to/from other components of drug delivery device 1000 (e.g. cartridge ID 770). Cartridge ID 770 may be any sort of mechanism or device that provides data about a component of drug delivery device 1000. For example, cartridge ID 770 may be a chip or RFID indicator on cartridge 220 (FIG. 12) that provides data regarding the type of liquid or medication within cartridge 220 (e.g. specific medication, viscosity, volume, dosage, injection scheduling, etc.). Other components, such as a needle assembly or motor, may include an ID to communicate other data related to drug delivery device 1000 (e.g. needle assembly type, needle length/positioning, patient information, historical treatment information, motor type, motor characteristics, etc.).

In some embodiments, drug delivery device 1000 may comprise an indicator (not shown) that provides some sort of indication that the medication has been delivered to the patient. Such an indication may be an end of dose indication. The indicator may comprise, for example, a light (e.g. an LED), a visual display such as a screen, a vibration, a sound, the sending of a signal, an indication on a separate computing device (e.g. a smartphone or computer as discussed above), a mechanical visual indicator (e.g. a window in the device housing to show barrel movement), or any combination thereof. The indicator may also indicate to a user any other information about the operation of drug delivery device 1000 including, but not limited to, whether or not a cartridge is inserted, whether a cartridge is inserted properly, device power information (e.g. whether the device is on/off, battery level), patient information, any information that may be provided by ID's discussed above, or any combination thereof.

In some embodiments, drug delivery device 1000 may comprise a number of sensors (not shown) that sense information related to the device. In an exemplary embodiment, drug delivery device 1000 comprises a skin sensor which senses whether the device is properly positioned against a patient's skin. In some embodiments, the drug delivery device 1000 may not actuate a needle assembly or deliver a medication if the skin sensor does not indicate that the device is positioned properly. Other examples of optional sensors include, but are not limited to, a medication level sensor, a pressure sensor, accelerometers, a force/thrust sensor, a position sensor for elements of the driving system, cartridge, housing, and/or needle assembly, a pH sensor, or any combination thereof.

The terms "first", "second", "third" and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the embodiments of the disclosure described herein are capable of operation in other sequences and/or arrangements than are described or illustrated herein.

While this invention has been described as having exemplary designs, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

Various aspects are described in this disclosure, which include, but are not limited to, the following aspects:
1. A drug delivery device including: a container containing a medication; a needle assembly coupled to the container, the needle assembly comprising: a housing with a first end and a second end configured to interface with the container; a first needle support and a second needle support positioned within the housing; a first needle coupled to the first needle support, a second needle coupled to the second needle support, a movable connector in fluid communication between the first and second needles; and a driving mechanism configured to move the needle assembly from a first configuration to a second configuration, wherein in the first configuration the first needle and the second needle are positioned completely within the housing, and in the second configuration the first needle extends beyond the first end of the housing and the second needle extends beyond the second end of the housing; and a delivery mechanism coupled to the container and configured to drive the medication through the second needle and the first needle when the needle assembly is in the second configuration.
2. The drug delivery device of aspect 1, wherein the container is configured to be decoupled from the needle assembly.
3. The drug delivery device of any one of aspects 1-2, wherein the movable connector is flexible.
4. The drug delivery device of aspect 3, wherein the movable connector is a flexible tube.
5. The drug delivery device of any one of aspects 1-4, wherein the driving mechanism is configured to operate at multiple different operating speeds.
6. The drug delivery device of any one of aspects 1-5, wherein each of the first needle support and the second needle support comprise a rack, and the driving mechanism is a pinion gear configured to drivably engage with the rack of the first needle support and the second needle support, respectively.
7. The drug delivery device of any one of aspects 1-6, the driving mechanism comprises a drive screw in threaded engagement with the first needle support and the second needle support.
8. The drug delivery device of any one of aspects 1-7, wherein the first end of the housing comprises a seal and the container comprises a septum proximate the second end of the housing, and when moving from the first configuration to the second configuration, the first needle pierces the seal, and the second needle pierces the septum.
9. The drug delivery device of any one of aspects 1-8, wherein the driving mechanism is further configured to move the needle assembly from the second configuration back to the first configuration.
10. The drug delivery device of any one of aspects 1-9 further comprising an electric motor, the electric motor configured to activate at least one of the delivery mechanism and the driving mechanism.
11. The drug delivery device of any one of aspects 1-10, further comprising a communication device configured to send and receive signals related to operation of the drug delivery device to and from a computing device.
12. A needle assembly including: a housing configured to couple to a fluid delivery mechanism and to interface with a surface; a first needle coupled to a first needle support; a second needle coupled to second needle support; a movable connector coupling the first needle to the second needle; and a driving mechanism supported by the housing and configured to drive the first needle support in a first direction, and to drive the second needle support in a second direction.
13. The needle assembly of aspect 12, further comprising a first motor configured to drive a fluid through the fluid delivery mechanism, and a second motor configured to actuate the driving mechanism.
14. The needle assembly of aspect 13, wherein the first and second motors are configured to operate with variable force.
15. The needle assembly of any one of aspects 12-14, wherein both the first needle support and the second needle support comprise at least one engagement feature configured to engage with the driving mechanism.
16. The needle assembly of aspect15, wherein the at least one engagement feature is a recess and the driving mechanism comprises a protrusion configured to interface with the recess, wherein the first and second directions are along a first axis and the at least one engagement feature is defined about a second axis orthogonal to the first axis.
17. The needle assembly of aspect 15, wherein the at least one engagement feature is a screw thread, the driving mechanism is a driving screw, and rotation of the driving screw moves the first needle support in the first direction and the second needle support in the second direction, wherein the first and second directions are along a first axis and the at least one engagement feature is defined about a second axis parallel to the first axis.
18. The needle assembly of any one of aspects 12-17, wherein the first direction and the second direction are both approximately co-linear and approximately opposite to one another.
19. The needle assembly of any one of aspects 12-18, wherein the movable connector is configured to flex in response to movement of at least one of the first needle support and the second needle support relative to one another.
20. The needle assembly of aspect 19, wherein the movable connector comprises a flexible tube configured to maintain a fluid coupling between the first needle and the second needle throughout movement of the first needle support and the second needle support.
21. The needle assembly of any one of aspects 12-20, wherein the fluid delivery mechanism is configured to deliver a variable amount of a fluid to a patient.
22. A method of operation of a device comprising the steps of: activating a first driving mechanism of the device a first time, wherein the first driving mechanism moves a first needle in a first direction from a retracted configuration to an axially extended configuration, and moves a second needle in a second direction towards a septum of a drug container; activating a second driving mechanism, wherein the second driving mechanism drives a medication through the second needle out of and the first needle; and activating the first driving mechanism a second time subsequent to activating the second drive mechanism, wherein the first driving mechanism moves the first needle in the second direction from the axially extended configuration to the retracted configuration and moves the second needle in the first direction away from the septum.
23. The method of aspect 22, wherein the first needle and the second needle are fluidly coupled with a flexible connector throughout each step.
24. The method of any one of aspects 22-23, wherein the first and the second driving mechanisms are activated by at least one electric motor.
25. The method of any one of aspects 22-24, further comprising the step of coupling the drug container to the needle assembly prior to the activating the first drive mechanism step.
26. The method of aspect 25, further comprising the step of decoupling the drug container from the needle assembly after activating the first driving mechanism the second time.
27. The method of any one of aspects 22-26, further comprising the step of increasing an index for a needle counter after activating the first driving mechanism the first time.
28. A needle assembly comprising: a housing configured to interface with a surface at a first end, and to interface with a container at a second end; a plurality of needle mechanisms, each needle mechanism comprising a first needle fluidly coupled to a second needle; at least one driving mechanism configured to engage at least one of the needle mechanisms wherein the first needle is driven in a first direction and the second needle is driven in a second direction approximately opposite to the first direction; and a rotating mechanism configured to rotate the plurality of needle mechanisms within the housing.
29. The needle assembly of aspect 28, wherein the rotating mechanism comprises a driven gear extending at least partially beyond the housing and configured to interface with a motor.
30. The needle assembly of any one of aspects 28-29, wherein the rotating mechanism is configured to position the first needle of one of the needle mechanisms near the surface, and the second needle of that needle mechanism near the container.
31. The needle assembly of aspect 30, wherein the first direction is towards the first end of the housing, and the second direction is towards the second end of the housing.
32. A drug delivery device comprising: a housing; at least one container within the housing configured to retain a fluid; a first motor configured to drive the fluid from the container with variable force; and a needle assembly configured to move from a first configuration to a second configuration, wherein in the first configuration the needle assembly is retracted, and in the second configuration the needle assembly is extended and in fluid communication with the container.
33. The drug delivery device of aspect 32, wherein the first motor moves the needle assembly from the first configuration to the second configuration.
34. The drug delivery device of any one of aspects 32-33, further comprising a second motor configured to move the needle assembly from the first configuration to the second configuration.
35. The drug delivery device of any one of aspects 32-34, further comprising an indicator configured to signal a user when the fluid is driven through the needle assembly.
36. The drug delivery device of any one of aspects 32-35, further comprising a first needle and a second needle within the needle assembly, wherein the first and second needles move along a first and second axis respectively when the needle assembly moves from the first configuration to the second configuration.
37. The drug delivery device of aspect 36, wherein the first and second axes are generally parallel.
38. The drug delivery device of aspect 36 wherein the first and second axes are generally orthogonal.
39. The drug delivery device of any one of aspects 32-38, further comprising a skin sensor configured to indicate whether the drug delivery device is appropriately positioned against a skin of a patient.

The claims of the parent application are reproduced immediately below as clauses. These clauses define preferred embodiments. The applicant reserves the right to pursue protection for subject-matter contained in the parent application as filed, either in the present divisional application or in a further application divided from the present divisional application.
1. A drug delivery device comprising:
   a container comprising a medication;
   a needle assembly coupled to the container, the needle assembly comprising:
      a housing with a first end and a second end configured to interface with the container;
      a first needle support and a second needle support positioned within the housing;
      a first needle coupled to the first needle support,
      a second needle coupled to the second needle support,
      a movable connector in fluid communication between the first and second needles; and
      a driving mechanism configured to move the needle assembly from a first configuration to a second configuration, wherein in the first configuration the first needle and the second needle are positioned completely within the housing, and in the second configuration the first needle extends beyond the first end of the housing and the second needle extends beyond the second end of the housing; and
   a delivery mechanism coupled to the container and configured to drive the medication through the second needle and the first needle when the needle assembly is in the second configuration.
2. The drug delivery device of clause 1, wherein the container is configured to be decoupled from the needle assembly.
3. The drug delivery device of any one of clauses 1-2, wherein the movable connector is flexible.
4. The drug delivery device of clause 3, wherein the movable connector is a flexible tube.
5. The drug delivery device of any one of clauses 1-4, wherein the driving mechanism is configured to operate at multiple different operating speeds.
6. The drug delivery device of any one of clauses 1-5, wherein each of the first needle support and the second needle support comprise a rack, and the driving mechanism is a pinion gear configured to drivably engage with the rack of the first needle support and the second needle support, respectively.
7. The drug delivery device of any one of clauses 1-6, the driving mechanism comprises a drive screw in threaded engagement with the first needle support and the second needle support.
8. The drug delivery device of any one of clauses 1-7, wherein the first end of the housing comprises a seal and the container comprises a septum proximate the second end of the housing, and when moving from the first configuration to the second configuration, the first needle pierces the seal, and the second needle pierces the septum.
9. The drug delivery device of any one of clauses 1-8, wherein the driving mechanism is further configured to move the needle assembly from the second configuration back to the first configuration.
10. The drug delivery device of any one of clauses 1-9 further comprising an electric motor, the electric motor configured to activate at least one of the delivery mechanism and the driving mechanism.
11. The drug delivery device of any one of clauses 1-10, further comprising a communication device configured to send and receive signals related to operation of the drug delivery device to and from a computing device.
12. The drug delivery device of any one of clauses 1-11, wherein during movement of the needle assembly from the first configuration to the second configuration, the first needle moves along an axis in a first direction and the second needle moves along an axis in a second direction opposite the first direction.
13. The drug delivery device of any one of clauses 1-11, wherein during movement of the needle assembly from the first configuration to the second configuration, the first needle moves along an axis in a first direction and the second needle moves along an axis in a second direction orthogonal to the first direction.
14. A needle assembly comprising:
   a housing configured to couple to a fluid delivery mechanism and to interface with a surface;
   a first needle coupled to a first needle support;
   a second needle coupled to a second needle support;
   a movable connector coupling the first needle to the second needle; and
   a driving mechanism supported by the housing and configured to drive the first needle support in a first direction, and to drive the second needle support in a second direction.
15. The needle assembly of clause 14, further comprising a first motor configured to drive a fluid through the fluid delivery mechanism, and a second motor configured to actuate the driving mechanism.
16. The needle assembly of clause 15, wherein the first and second motors are configured to operate with variable force.
17. The needle assembly of any one of clauses 14-16, wherein both the first needle support and the second needle support comprise at least one engagement feature configured to engage with the driving mechanism.
18. The needle assembly of clause 17, wherein the at least one engagement feature is a recess and the driving mechanism comprises a protrusion configured to interface with the recess, wherein the first and second directions are along a first axis and the at least one engagement feature is defined about a second axis orthogonal to the first axis.
19. The needle assembly of clause 17, wherein the at least one engagement feature is a screw thread, the driving mechanism is a driving screw, and rotation of the driving screw moves the first needle support in the first direction and the second needle support in the second direction, wherein the first and second directions are along a first axis and the at least one engagement feature is defined about a second axis parallel to the first axis.
20. The needle assembly of any one of clauses 14-19, wherein the first direction and the second direction are both approximately co-linear and approximately opposite to one another.
21. The needle assembly of any one of clauses 14-19, wherein the first direction and the second direction are approximately orthogonal to one another.
22. The needle assembly of any one of clauses 14-21, wherein the movable connector is configured to flex in response to movement of at least one of the first needle support and the second needle support relative to one another.
23. The needle assembly of clause 22, wherein the movable connector comprises a flexible tube configured to maintain a fluid coupling between the first needle and the second needle throughout movement of the first needle support and the second needle support.
24. The needle assembly of any one of clauses 14-23, wherein the fluid delivery mechanism is configured to deliver a variable amount of a fluid to a patient.
25. A method of operation of a device comprising the steps of:
   activating a first driving mechanism of the device a first time, wherein the first driving mechanism moves a first needle in a first direction from a retracted configuration to an axially extended configuration, and moves a second needle in a second direction towards a septum of a drug container;
   activating a second driving mechanism, wherein the second driving mechanism drives a medication through the second needle out of and the first needle; and
   activating the first driving mechanism a second time subsequent to activating the second drive mechanism, wherein the first driving mechanism moves the first needle in the second direction from the axially extended configuration to the retracted configuration and moves the second needle in the first direction away from the septum.
26. The method of clause 25, wherein the first needle and the second needle are fluidly coupled with a flexible connector throughout each step.
27. The method of any one of clauses 25-26, wherein the first and the second driving mechanisms are activated by at least one electric motor.
28. The method of any one of clauses 25-27, further comprising the step of coupling the drug container to the needle assembly prior to the activating the first drive mechanism step.
29. The method of clause 28, further comprising the step of decoupling the drug container from the needle assembly after activating the first driving mechanism the second time.
30. The method of any one of clauses 25-29, further comprising the step of increasing an index for a needle counter after activating the first driving mechanism the first time.
31. A needle assembly comprising:
   a housing configured to interface with a surface at a first end, and to interface with a container at a second end;
   a plurality of needle mechanisms, each needle mechanism comprising a first needle and a second needle fluidly coupled to the first needle;
   at least one driving mechanism configured to engage at least one of the needle mechanisms wherein the first needle is driven in a first direction and the second needle is driven in a second direction approximately opposite to the first direction; and
   a rotating mechanism configured to rotate the plurality of needle mechanisms within the housing.
32. The needle assembly of clause 31, wherein the rotating mechanism comprises a driven gear extending at least partially beyond the housing and configured to interface with a motor.
33. The needle assembly of any one of clauses 31-32, wherein the rotating mechanism is configured to position the first needle of one of the needle mechanisms near the surface, and the second needle of that needle mechanism near the container.
34. The needle assembly of clause 33, wherein the first direction is towards the first end of the housing, and the second direction is towards the second end of the housing.
35. A drug delivery device comprising:
   a housing;
   at least one container within the housing configured to retain a fluid;
   a first motor configured to drive the fluid from the container with variable force; and
   a needle assembly configured to move from a first configuration to a second configuration, wherein in the first configuration the needle assembly is retracted, and in the second configuration the needle assembly is extended and in fluid communication with the container.
36. The drug delivery device of clause 35, wherein the first motor moves the needle assembly from the first configuration to the second configuration.
37. The drug delivery device of any one of clauses 35-36, further comprising a second motor configured to move the needle assembly from the first configuration to the second configuration.
38. The drug delivery device of any one of clauses 35-37, further comprising an indicator configured to signal a user when the fluid is driven through the needle assembly.
39. The drug delivery device of any one of clauses 35-38, further comprising a first needle and a second needle within the needle assembly, wherein the first and second needles move along a first and second axis respectively when the needle assembly moves from the first configuration to the second configuration.
40. The drug delivery device of clause 39, wherein the first and second axes are generally parallel.
41. The drug delivery device of clause 39 wherein the first and second axes are generally orthogonal.
42. The drug delivery device of any one of clauses 35-41, further comprising a skin sensor configured to indicate whether the drug delivery device is appropriately positioned against a skin of a patient.

## Claims

1. A needle mechanism comprising:
a first needle coupled to a first needle support;
a second needle coupled to a second needle support;
a movable connector coupling the first needle to the second needle; and
a driving mechanism configured to rotate and drive the first needle support in a first direction and the second needle support in a second direction.

2. The needle mechanism of claim 1, wherein both the first needle support and the second needle support comprise at least one engagement feature configured to engage with the driving mechanism.

3. The needle mechanism of claim 2, wherein the at least one engagement feature is a recess and the driving mechanism comprises a protrusion configured to interface with the recess, wherein the first and second directions are along a first axis and the at least one engagement feature is defined about a second axis orthogonal to the first axis.

4. The needle mechanism of claim 2, wherein the at least one engagement feature is a screw thread, the driving mechanism is a driving screw, and rotation of the driving screw moves the first needle support in the first direction and the second needle support in the second direction, wherein the first and second directions are along a first axis and the at least one engagement feature is defined about a second axis parallel to the first axis.

5. The needle mechanism of any one of claims 1-2, wherein the first direction and the second direction are both approximately co-linear and approximately opposite to one another.

6. The needle mechanism of any one of claims 1-2, wherein the first direction and the second direction are approximately orthogonal to one another.

7. The needle mechanism of any one of claims 1-6, wherein the movable connector is configured to flex in response to movement of at least one of the first needle support and the second needle support relative to one another.

8. The needle mechanism of claim 7, wherein the movable connector comprises a flexible tube configured to maintain a fluid coupling between the first needle and the second needle throughout movement of the first needle support and the second needle support.

9. The needle mechanism of any one of claims 1-3 and 5-8, wherein each of the first needle support and the second needle support comprises a rack, and the driving mechanism is a pinion gear configured to drivably engage with the rack of the first needle support and the second needle support, respectively.

10. The needle mechanism of any one of claims 1-2, 4-6, and 7-9, the driving mechanism comprises a drive screw in threaded engagement with the first needle support and the second needle support.

11. The needle mechanism of any one of claims 1-10, the driving mechanism is configured to rotate and drive the first needle support in the second direction, and to drive the second needle support in the first direction.

12. A needle assembly comprising:
the needle mechanism of any preceding claim; and
a motor configured to actuate rotation of the driving mechanism.

13. The needle assembly of claim 12, wherein the motor is configured to operate with variable force.
